# EUROPEAN PATENT APPLICATION

(11) **EP 4 725 940 A1**
(43) Date of publication of application: **15.04.2026**
(21) Application number: 24838653.4
(22) Date of filing: 03.07.2024
(51) Int. Cl.: C07D 207/448, C07D 401/06, C07D 409/06, A61K 31/4015, A61K 31/4025, A61K 31/4436, A61P 35/00, A61P 35/02, A61P 3/10, A61P 37/06

(54) **HISTONE DEACETYLASE INHIBITOR AND USE**

(30) Priority: 11.07.2023 CN 202310842593
(71) Applicant: Nanjing Hongshun Pharmaceutical Technology Co., Ltd, Nanjing, Jiangsu 210000 (CN); China Pharmaceutical University, Nanjing, Jiangsu 210000 (CN)
(72) Inventor: ZHOU, Qingfa, Nanjing, Jiangsu 210000 (CN); YAO, Shengfa, Nanjing, Jiangsu 210000 (CN); TANG, Yujiang, Nanjing, Jiangsu 210000 (CN)
(74) Representative: Bayramoglu et al.
(86) International application number: PCT/CN2024/103237
(87) International publication number: WO 2025/011398

(57) **Abstract**

The present invention relates to a histone deacetylase inhibitor and uses thereof, belonging to the field of medicinal chemistry. The present invention prepares a class of HDAC inhibitors with a novel structure and high efficiency by introducing a succinimide fragment. The present invention has the beneficial effects of excellent inhibitory activity against HDACs and excellent in vitro anti-tumor activity, providing a new approach for the application of HDAC inhibitors in solid tumors and/or the treatment of diseases associated with uncontrolled histone deacetylase activity.

## Description

### TECHNICAL FIELD

The present invention relates to a histone deacetylase inhibitor and uses thereof, belonging to the field of chemistry.

### BACKGROUND TECHNOLOGY

Epigenetic abnormalities are closely related to the occurrence of various human diseases, and they are mainly caused by the dysfunction of epigenetic proteins due to gene deficiency. Histone deacetylases (HDACs) and histone acetyl transferases (HATs), a pair of enzymes with opposing biological functions, dynamically regulate the deacetylation and acetylation of histones. In normal cells, HDACs and HATs can maintain a dynamic balance, which is crucial for the normal expression of genes. This dynamic balance between HDACs and HATs is generally disrupted by the abnormally enhanced biological activity of HDACs, leading to abnormal expression of genes related to cell cycle and apoptosis, as well as abnormalities in corresponding signaling pathways. Studies have shown that the abnormal expression of HDACs is closely related to the occurrence and development of various diseases such as cancer, inflammation and neurological diseases. Therefore, the development of HDAC inhibitors has aroused widespread interest in the field of drug development and exhibited broad application prospects. Up to now, the U. S. Food and Drug Administration has successively approved four HDAC inhibitors, namely, vorinostat, romidepsin, belinostat and panobinostat, as well as chidamide approved by the China Food and Drug Administration. They are mainly used to treat hematological cancers such as cutaneous T-cell lymphoma, peripheral T-cell lymphoma, and multiple myeloma. However, the HDAC inhibitors face significant bottlenecks and challenges as follows: the currently available HDAC inhibitors have been successfully used in the treatment of hematological cancers, but showing less satisfactory efficacy in monotherapies for solid tumors, such as breast cancer, renal cancer, prostate cancer and head and neck cancer, limiting their widespread application in clinical practice. Therefore, there is an urgent clinical need to develop an HDAC inhibitor with novel structure and high efficiency and to broaden its application in solid tumors.

### CONTENT OF THE INVENTION

In view of the defect of poor therapeutic effect of HDAC inhibitors on solid tumors in the prior art, an object of the present invention is to develop a new histone deacetylase inhibitor, aiming to provide the HDAC inhibitor with higher efficiency and safety and improve the therapeutic effect of the HDAC inhibitor on solid tumors.

An HDAC small molecule inhibitor mainly consists of four components, including a zinc binding group (ZBG), a linker, a connect unit (CU) and a cap group. In the prior art, studies on the HDAC small molecule inhibitor mainly focus on ZBG and CU, and in most cases, the CU of most HDAC inhibitors is a simple sp2 hybrid group, for example, amide and carbonyl. However, CU is crucial for molecular conformation and thus for efficacy and pharmacokinetic properties. Therefore, an object of the present invention is to provide a class of novel and effective HDAC inhibitors and pharmaceutically acceptable salts thereof. Based on this object, the present invention first provides the following compound of a formula below: 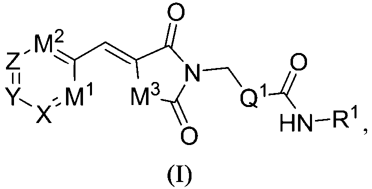 wherein in the formula, X, Y, Z, M¹, and M² each independently represent a carbon or nitrogen atom;
in a case where X, Y, Z, M¹, and M² each independently represent the carbon atom, X, Y, Z, M¹, and M² are each independently and optionally substituted by R², with R² being hydrogen, alkyl, cyano, halogen, haloalkyl, hydroxy, mercapto, alkoxy, alkylamino, alkylthio, alkoxyalkyl, arylalkyl, diarylalkyl, aryl, or Het;
M³ is carbon and may be independently and optionally substituted by R⁴ or R⁵, with R⁴ or R⁵ being hydrogen, deuterium, alkyl, haloalkyl, hydroxy, mercapto, alkoxy, alkylamino, alkylthio, alkoxyalkyl, methylene, arylalkyl, diarylalkyl, aryl, or Het;
Q¹ is selected from saturated or unsaturated linear or branched hydrocarbonyl, aryl, or Het with a length of 1-8 carbon atoms;
R¹ is selected from hydroxy, or 2-aminophenyl substituted by one or more R³;
R³ is hydrogen, alkyl, cyano, halogen, haloalkyl, hydroxy, mercapto, alkoxy, alkylthio, alkoxyalkyl, arylalkyl, aryl, or Het;
the alkyl is linear or branched saturated hydrocarbonyl with 1-6 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms;
the alkoxy is linear or branched saturated hydrocarbonyl with 1-6 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms, wherein each carbon atom is optionally substituted by oxygen;
the alkylamino is linear or branched saturated hydrocarbonyl with 1-6 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms, wherein each carbon atom is optionally substituted by an NH atomic group;
the alkoxyalkyl is derived by linking the alkoxy to the alkyl as defined above;
alkenyl or alkynyl is linear or branched doubled-bonded or triple-bonded unsaturated hydrocarbonyl with 1-6 carbon atoms;
the aryl is a carbocyclic ring selected from phenyl, naphthyl, acenaphthenyl, or tetrahydronaphthyl, and is each optionally substituted by 1, 2, or 3 substituents, with each substituent independently selected from hydrogen, alkyl, cyano, halogen, haloalkyl, hydroxy, mercapto, alkoxy, alkylthio, alkoxyalkyl, arylalkyl, diarylalkyl, aryl, or Het;
the arylalkyl or the diarylalkyl is derived by linking the aryl to the alkyl as defined above;
the Het is a monocyclic heterocyclic ring selected from pyrrolyl, pyrazolyl, imidazolyl, furyl, thienyl, oxazolyl, isooxazolyl, thiazolyl, isothiazolyl, pyridyl, pyrimidinyl, pyrazinyl, or pyridazinyl, or a bicyclic heterocyclic ring selected from quinolyl, quinoxalinyl, indolyl, benzoimidazolyl, benzoxazolyl, benzoisooxazolyl, benzothiazolyl, benzoisothiazolyl, benzofuryl, benzothienyl, 2, 3-dihydrobenzo[1,4]dioxacyclohexenyl, or benzo[1,3]dioxacyclopentenyl, or is selected from monocyclic saturated hydrocarbonyl with 3-6 carbon atoms, or bicyclic saturated hydrocarbonyl with 6-12 carbon atoms, wherein the carbon atom in the ring is independently substituted by 1-4 O, S, N, or NH, each monocyclic or bicyclic ring is optionally substituted by 1, 2, or 3 substituents, and each substituent is independently selected from halogen, haloalkyl, hydroxy, alkyl, or alkoxy;
the halogen is a substituent selected from fluorine, chlorine, bromine, or iodine; and
the haloalkyl is linear or branched saturated hydrocarbonyl with 1-6 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms, wherein one or more carbon atoms are optionally substituted by one or more halogen atoms.

The present invention further selects the following compound as an HDAC inhibitor: in the formula, X, Y, Z, M¹, and M² each independently represent a carbon or nitrogen atom, and in a case where X, Y, Z, M¹, and M² each independently represent the carbon atom, X, Y, Z, M¹, and M² are each independently and optionally substituted by R², with R² being hydrogen, alkyl, cyano, halogen, haloalkyl, alkoxy, alkylthio, or alkoxyalkyl;
M³ is carbon and may be independently and optionally substituted by R⁴ or R⁵, with R⁴ or R⁵ being hydrogen, deuterium, alkyl, haloalkyl, hydroxy, mercapto, alkoxy, alkylamino, alkylthio, alkoxyalkyl, methylene, arylalkyl, diarylalkyl, aryl, or Het;
Q¹ is selected from saturated or unsaturated linear or branched hydrocarbonyl or aryl with a length of 1-8 carbon atoms;
R¹ is selected from hydroxy, or 2-aminophenyl optionally substituted by one or more R³; and
R³ is hydrogen, alkyl, cyano, halogen, haloalkyl, aryl, or Het.

The present invention further selects the following compound as an HDAC inhibitor: in the formula, X, Y, Z, M¹, and M² each independently represent a carbon or nitrogen atom, and in a case where X, Y, Z, M¹, and M² each independently represent the carbon atom, X, Y, Z, M¹, and M² are each independently and optionally substituted by R², with R² being hydrogen, alkyl, halogen, or alkoxy;
M³ is carbon and may be independently and optionally substituted by R⁴ or R⁵, and R⁴ or R⁵ is hydrogen, deuterium, alkyl, haloalkyl, hydroxy, mercapto, alkoxy, alkylamino, alkylthio, alkoxyalkyl, methylene, arylalkyl, diarylalkyl, aryl, or Het;
Q¹ is selected from saturated or unsaturated linear or branched hydrocarbonyl or aryl with a length of 3-8 carbon atoms;
R¹ is selected from hydroxy, or 2-aminophenyl optionally substituted by one or more R³; and
R³ is hydrogen, alkyl, cyano, halogen, haloalkyl, aryl, or Het.

Then, the present invention further selects the following compound as an HDAC inhibitor:
(E)-4-(3-benzal-2,5-dioxopyrrolidinyl)-N-hydroxybutyramide (I-1),
(E)-4-(3-benzal-2,5-dioxopyrrolidinyl)-N-hydroxypentanamide (I-2),
(E)-4-(3-benzal-2,5-dioxopyrrolidinyl)-N-hydroxyhexanamide (I-3),
(E)-4-(3-benzal-2,5-dioxopyrrolidinyl)-N-hydroxyheptamide (I-4),
(E)-4-(3-benzal-2,5-dioxopyrrolidinyl)-N-hydroxydecoylamide (I-5),
(E)-N-(2-amino-4-fluorophenyl)-4-(3-benzal-2,5-diketopyrrolidinyl)butyramide (I-6),
(E)-N-(2-amino-4-fluorophenyl)-5-(3-benzal-2,5-diketopyrrolidinyl)pentanamide (I-7),
(E)-N-(2-amino-4-fluorophenyl)-6-(3-benzal-2,5-diketopyrrolidinyl)hexanamide (I-8),
(E)-N-(2-amino-4-fluorophenyl)-7-(3-benzal-2,5-diketopyrrolidinyl)heptamide (I-9),
4-(3-((E)-benzal)-2,5-diketopyrrolidinyl)-N-((1S,2S)-2-hydroxycyclohexyl)butyramide (I-10),
5-(3-((E)-benzal)-2,5-diketopyrrolidinyl)-N-((1S,2S)-2-hydroxycyclohexyl)pentanami de (I-11),
6-(3-((E)-benzal)-2,5-diketopyrrolidinyl)-N-((1S,2S)-2-hydroxycyclohexyl)hexanamid e (I-12),
7-(3-((E)-benzal)-2,5-diketopyrrolidinyl)-N-((1S,2S)-2-hydroxycyclohexyl) heptamide (I-13),
8-(3-((E)-benzal)-2,5-diketopyrrolidinyl)-N-((1S,2S)-2-hydroxycyclohexyl)decoylami de (I-14),
(E)-7-(3-(4-isopropylbenzal)-2,5-diketopyrrolidinyl)-N-hydroxyheptamide (I-15),
(E)-7-(3-(4-dimethylaminobenzal)-2,5-diketopyrrolidinyl)-N-hydroxyheptamide (I-16),
(E)-7-(3-(4-trifluoromethylbenzal)-2,5-diketopyrrolidinyl)-N-hydroxyheptamide (I-17),
(E)-7-(3-(4-methylbenzal)-2,5-diketopyrrolidinyl)-N-hydroxyheptamide (I-18),
(E)-7-(3-(2-methylbenzal)-2,5-diketopyrrolidinyl)-N-hydroxyheptamide (I-19),
(E)-7-(3-(2-cyanobenzal)-2,5-diketopyrrolidinyl)-N-hydroxyheptamide (I-20),
(E)-7-(3-(2-methylenepyridin)-2,5-diketopyrrolidinyl)-N-hydroxyheptamide (I-21),
(E)-7-(3-(2-methylenenaphthyl)-2,5-diketopyrrolidinyl)-N-hydroxyheptamide (I-22),
(E)-7-(3-(2-methylenethienyl)-2,5-diketopyrrolidinyl)-N-hydroxyheptamide (I-23),
(E)-7-(3-(2-chlorobenzal)-2,5-diketopyrrolidinyl)-N-hydroxyheptamide (I-24),
(E)-7-(3-(3-chlorobenzal)-2,5-diketopyrrolidinyl)-N-hydroxyheptamide (I-25),
(E)-7-(3-(4-chlorobenzal)-2,5-diketopyrrolidinyl)-N-hydroxyheptamide (I-26),
(E)-7-(3-(3-bromobenzal)-2,5-diketopyrrolidinyl)-N-hydroxyheptamide (I-27),
(E)-7-(3-(3-fluorobenzal)-2,5-diketopyrrolidinyl)-N-hydroxyheptamide (I-28),
(E)-7-(3-(3-iodobenzal)-2,5-diketopyrrolidinyl)-N-hydroxyheptamide (I-29),
(E)-7-(3-(3,4-dichlorobenzal)-2,5-diketopyrrolidinyl)-N-hydroxyheptamide (I-30),
(E)-7-(3-(2-methoxybenzal)-2,5-diketopyrrolidinyl)-N-hydroxyheptamide (I-31),
(E)-7-(3-(3-methoxybenzal)-2,5-diketopyrrolidinyl)-N-hydroxyheptamide (I-32),
(E)-7-(3-(4-methoxybenzal)-2,5-diketopyrrolidinyl)-N-hydroxyheptamide (I-33),
(E)-7-(3-(3,4-dimethoxybenzal)-2,5-diketopyrrolidinyl)-N-hydroxyheptamide (I-34),
(E)-7-(3-(3,4,5-trimethoxybenzal)-2,5-diketopyrrolidinyl)-N-hydroxyheptamide (I-35),
(E)-7-(3-(4-hydroxy-3,5-dimethoxybenzal)-2,5-diketopyrrolidinyl)-N-hydroxyheptami de (I-36),
(E)-7-(3-(2-hydroxybenzal)-2,5-diketopyrrolidinyl)-N-hydroxyheptamide (I-37),
(E)-7-(3-(2-ethoxybenzal)-2,5-diketopyrrolidinyl)-N-hydroxyheptamide (I-38),
(E)-7-(3-(5-bromo-2-methoxybenzal)-2,5-diketopyrrolidinyl)-N-hydroxyheptamide (I-39),
(E)-7-(3-(4-bromo-2-methoxybenzal)-2,5-diketopyrrolidinyl)-N-hydroxyheptamide (I-40), or
(E)-4-((3-benzal-2,5-diketopyrrolidinyl)methyl)-N-hydroxybenzamide (I-41).

The compounds described above are prepared according to the following method: 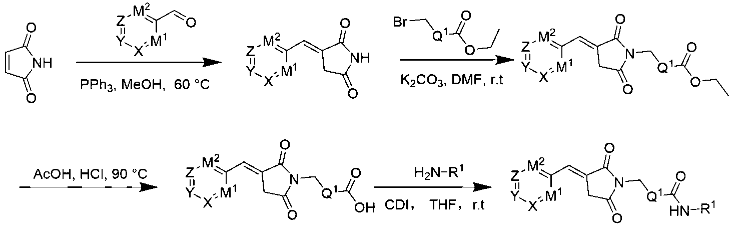 The compounds of the present invention are prepared according to the above or similar preparation method by selecting corresponding starting materials depending on the substituents and the positions of the substitutents.

The present invention further provides a pharmaceutical composition, which includes the compound described above or the pharmaceutically acceptable salt thereof, and a pharmaceutically acceptable carrier, wherein the compound described above or the pharmaceutically acceptable salt thereof is an active ingredient.

The present invention further provides a use of the above compound in preparation of a medicament for preventing or treating an HDAC-related clinical disease. Here, the HDAC-related clinical disease includes lung cancer, melanoma, liver cancer, kidney cancer, leukemia, prostate cancer, thyroid cancer, skin disease, pancreatic cancer, ovarian cancer, testicular cancer, breast cancer, bladder cancer, gallbladder cancer, myelodysplastic syndrome, lymphoma, esophageal cancer, gastrointestinal cancer, astrocytoma, neuroblastoma, glioma, schwannoma, mesothelioma, non-insulin-dependent diabetes mellitus, and autoimmune disease.

The present invention prepares a class of HDAC inhibitors with a novel structure and high efficiency by cyclization of an amide bond and CU to form a five-membered ring. The present invention has the beneficial effects of excellent inhibitory activity against HDACs and excellent in vitro anti-tumor activity, providing a new approach for studying the application of HDAC inhibitors in solid tumors.

### SPECIFIC IMPLEMENTATIONS

### Example 1

### (E)-4-(3-benzal-2,5-dioxopyrrolidinyl)-N-hydroxybutyramide (I-1)

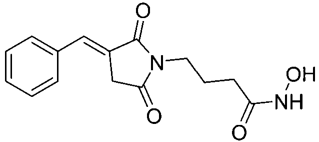

### Step a: Synthesis of (E)-3-benzalpyrrolidin-2,5-dione

Maleimide (5 mmol, 485.35 mg), methanol (10 mL), triphenylphosphine (6 mmol, 1.57 g) and benzaldehyde (7.5 mmol, 795 mg) were added to a 100 mL round-bottom flask in sequence, and refluxed at 60°C under stirring to react for 8 h. A reaction mixture was cooled to room temperature for solid precipitation and then subjected to suction filtration, and a filter cake was dried to obtain 750 mg of white solids, with the yield of 80.1%. 1H NMR (400 MHz, DMSO-d6) δ 11.45 (s, 1H), 7.66 - 7.59 (m, 2H), 7.52 - 7.41 (m, 3H), 7.39 (t, J = 2.5 Hz, 1H), 3.66 (d, J = 2.5 Hz, 2H).

### Step b: Synthesis of (E)-4-(3-benzal-2,5-dioxopyrrolidinyl)ethyl butyrate

(E)-3-benzalpyrrolidin-2,5-dione (749 mg, 4 mmol), DMF (10 mL) and potassium carbonate (829 mg, 6 mmol) were added to a 100 mL round-bottom flask in sequence and then stirred in an ice bath for 30 min; after the ice bath was removed and the reaction mixture returned to normal temperature, ethyl bromobutyrate (1.56 g, 8 mmol) was added; and the mixture was stirred for 8 h at normal temperature. The mixture was extracted with water and ethyl acetate, and an organic layer was concentrated, eluted by column chromatography using a mixed solvent of petroleum ether and ethyl acetate at a volume ratio of 3:1 as an eluent, and then separated to obtain 678 mg of transparent liquid, with the yield of 56.2%. 1H NMR (300 MHz, Chloroform-d) δ 7.71 - 7.36 (m, 6H), 4.13 (q, J = 7.1 Hz, 2H), 3.70 (t, J = 6.9 Hz, 2H), 3.57 (d, J = 2.4 Hz, 2H), 2.36 (t, J = 7.4 Hz, 2H), 1.99 (p, J = 7.2 Hz, 2H), 1.26 (t, J = 7.1 Hz, 3H).

### Step c: Synthesis of (E)-4-(3-benzal-2,5-dioxopyrrolidinyl)-N-hydroxybutyramide

(E)-4-(3-benzal-2,5-dioxopyrrolidinyl)ethyl butyrate (600 mg, 1.99 mmol), 3 mL of glacial acetic acid and 0.5 mL of concentrated hydrochloric acid were added to a 50 mL round-bottom flask and stirred at 90°C for reaction; after TLC monitoring indicated complete reaction, the reaction mixture was cooled and added to water for solid precipitation; filtration was carried out to collect a filter cake; and the filter cake was dried.

The dried filter cake was added to a 50 mL round-bottom flask, and dissolved with 5 mL of tetrahydrofuran; 484 mg of N,N-carbonyldiimidazole was added and stirred to react; 1 h later, hydroxylamine hydrochloride (415 mg, 5.97 mmol) was added slowly and stirred to react for 12 h; the reaction mixture was concentrated, eluted by column chromatography using a mixed solvent of dichloromethane and methanol at a volume ratio of 25:1 as an eluent, and then separated to obtain 63 mg of light yellow solids, with the yield of 11%. 1H NMR (400 MHz, DMSO-d6) δ 10.36 (s, 1H), 8.71 (s, 1H), 7.69 - 7.62 (m, 2H), 7.48 (qt, J = 6.9, 3.7 Hz, 5H), 3.70 (d, J = 2.4 Hz, 2H), 3.50 (t, J = 7.1 Hz, 2H), 2.03 - 1.94 (m, 2H), 1.76 (p, J = 7.5 Hz, 2H).13C NMR (101 MHz, DMSO-d6) δ 174.78, 171.15, 168.83, 134.60, 132.37, 130.70, 130.27, 129.46, 125.80, 38.29, 34.20, 30.28, 24.05.

### Example 2

### (E)-4-(3-benzal-2,5-dioxopyrrolidinyl)-N-hydroxypentanamide (I-2)

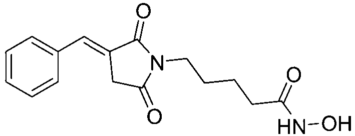

### Step a: Synthesis of (E)-3-benzalpyrrolidin-2,5-dione

The operation and feeding were the same as Step a in Example 1.

### Step b: Synthesis of (E)-5-(3-benzal-2,5-dioxopyrrolidinyl)ethyl pentanoate

The compound of interest was synthesized with a method similar to Step b in Example 1 by taking ethyl bromopentanoate as a starting material.

### Step c: Synthesis of (E)-5-(3-benzal-2,5-dioxopyrrolidinyl)-N-hydroxypentanamide

The compound of interest was synthesized with a method similar to Step c in Example 1 by taking (E)-5-(3-benzal-2,5-dioxopyrrolidinyl)ethyl pentanoate as a starting material. The compound of interest was a white solid, with the yield of 15.2%. 1H NMR (400 MHz, DMSO-d6) δ 10.36 (s, 1H), 8.71 (s, 1H), 7.69 - 7.61 (m, 2H), 7.53 - 7.40 (m, 4H), 3.72 (d, J = 2.4 Hz, 2H), 3.48 (t, J = 6.2 Hz, 2H), 1.97 (t, J = 6.6 Hz, 2H), 1.58 - 1.48 (m, 2H), 1.51 - 1.41 (m, 2H).

13C NMR (101 MHz, DMSO) δ 174.80, 171.16, 169.23, 134.59, 132.42, 130.71, 130.28, 129.45, 125.74, 38.29, 34.15, 32.28, 27.44, 23.00.

### Example 3

### (E)-4-(3-benzal-2,5-dioxopyrrolidinyl)-N-hydroxyhexanamide (I-3)

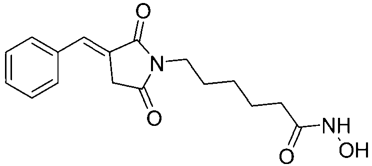

### Step a: Synthesis of (E)-3-benzalpyrrolidin-2,5-dione

The operation and feeding were the same as Step a in Example 1.

### Step b: Synthesis of (E)-6-(3-benzal-2,5-dioxopyrrolidinyl)ethyl hexanoate

The compound of interest was synthesized with a method similar to Step b in Example 1 by taking ethyl bromohexanoate as a starting material.

### Step c: Synthesis of (E)-6-(3-benzal-2,5-dioxopyrrolidinyl)-N-hydroxyhexanamide

The compound of interest was synthesized with a method similar to Step c in Example 1 by taking (E)-6-(3-benzal-2,5-dioxopyrrolidinyl)ethyl hexanoate as a starting material. The compound of interest was a yellow solid, with the yield of 18.2%. 1H NMR (400 MHz, DMSO-d6) δ 10.33 (s, 1H), 8.66 (s, 1H), 7.69 - 7.62 (m, 2H), 7.56 - 7.40 (m, 4H), 3.72 (d, J = 2.4 Hz, 2H), 3.48 (t, J = 7.2 Hz, 2H), 1.94 (t, J = 7.4 Hz, 2H), 1.52 (dqd, J = 15.6, 7.7, 0.0 Hz, 4H), 1.31 - 1.17 (m, 2H).

13C NMR (101 MHz, DMSO) δ 174.79, 171.15, 169.45, 134.58, 132.40, 130.70, 130.28, 129.45, 125.74, 38.43, 34.14, 32.52, 27.57, 26.33, 25.18.

### Example 4

### (E)-4-(3-benzal-2,5-dioxopyrrolidinyl)-N-hydroxyheptamide (I-4)

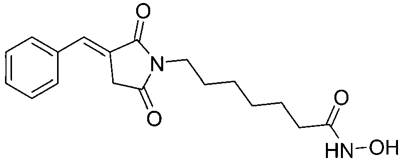

### Step a: Synthesis of (E)-3-benzalpyrrolidin-2,5-dione

The operation and feeding were the same as Step a in Example 1.

### Step b: Synthesis of (E)-7-(3-benzal-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-benzal-2,5-dioxopyrrolidinyl)-N-hydroxyheptamide

The compound of interest was synthesized with a method similar to Step c in Example 1 by taking ((E)-7-(3-benzal-2,5-dioxopyrrolidinyl)ethyl heptanoate as a starting material. The compound of interest was a white solid, with the yield of 16.1%. 1H NMR (400 MHz, DMSO-d6) δ 10.34 (s, 1H), 8.66 (s, 1H), 7.68 - 7.62 (m, 2H), 7.47 (qt, J = 6.9, 3.6 Hz, 4H), 3.71 (d, J = 2.4 Hz, 2H), 3.48 (t, J = 7.2 Hz, 2H), 1.94 (t, J = 7.4 Hz, 2H), 1.58 - 1.42 (m, 4H), 1.32 - 1.21 (m, 4H).

13C NMR (101 MHz, DMSO) δ 174.78, 171.15, 169.53, 134.60, 132.38, 130.70, 130.26, 129.44, 125.76, 38.48, 34.14, 32.64, 28.63, 27.67, 26.44, 25.45.

### Example 5

### (E)-4-(3-benzal-2,5-dioxopyrrolidinyl)-N-hydroxydecoylamide (I-5)

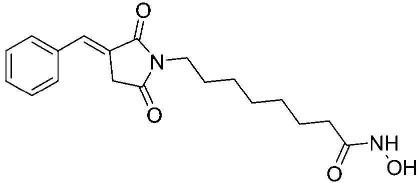

### Step a: Synthesis of (E)-3-benzalpyrrolidin-2,5-dione

The operation and feeding were the same as Step a in Example 1.

### Step b: Synthesis of (E)-8-(3-benzal-2,5-dioxopyrrolidinyl)ethyl heptanoate

The compound of interest was synthesized with a method similar to Step b in Example 1 by taking ethyl bromooctanoate as a starting material.

### Step c: Synthesis of (E)-8-(3-benzal-2,5-dioxopyrrolidinyl)-N-hydroxyheptamide

The compound of interest was synthesized with a method similar to Step c in Example 1 by taking (E)-8-(3-benzal-2,5-dioxopyrrolidinyl)ethyl heptanoate as a starting material. The compound of interest was a faint yellow solid, with the yield of 20.2%. 1H NMR (400 MHz, DMSO-d6) δ 10.33 (s, 1H), 8.66 (s, 1H), 7.71 - 7.61 (m, 2H), 7.53 - 7.40 (m, 4H), 3.72 (d, J = 2.4 Hz, 2H), 3.48 (t, J = 7.2 Hz, 2H), 1.93 (t, J = 7.3 Hz, 2H), 1.56 - 1.43 (m, 4H), 1.31 - 1.19 (m, 6H).

13C NMR (101 MHz, DMSO) δ 174.76, 171.15, 169.60, 134.58, 132.40, 130.68, 130.25, 129.43, 125.72, 38.50, 34.13, 32.71, 28.94, 28.78, 27.75, 26.65, 25.53.

### Example 6

### (E)-N-(2-amino-4-fluorophenyl)-4-(3-benzal-2,5-diketopyrrolidinyl)butyramide (I-6)

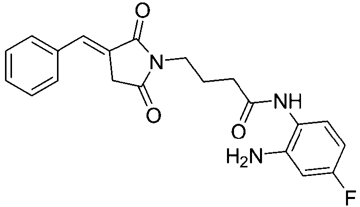

### Step a: Synthesis of (E)-3-benzalpyrrolidin-2,5-dione

The operation and feeding were the same as Step a in Example 1.

### Step b: Synthesis of (E)-4-(3-benzal-2,5-dioxopyrrolidinyl)ethyl butyrate

### The compound of interest was synthesized with a method similar to Step b in Example 1 by taking ethyl bromobutyrateas a starting material.

### Step c: Synthesis of (E)-N-(2-amino-4-fluorophenyl)-4-(3-benzal-2,5-diketopyrrolidinyl)butyramide

The compound of interest was synthesized with a method similar to Step c in Example 1 by taking (E)-4-(3-benzal-2,5-dioxopyrrolidinyl)ethyl butyrate and diamino-4-fluorobenzene as starting materials. The compound of interest was a white solid, with the yield of 25.2%. 1H NMR (400 MHz, DMSO-d6) δ 9.01 (s, 1H), 7.69 - 7.62 (m, 2H), 7.52 - 7.42 (m, 4H), 7.11 - 7.01 (m, 1H), 6.48 (dd, J = 11.2, 2.9 Hz, 1H), 6.27 (td, J = 8.6, 2.9 Hz, 1H), 5.18 (s, 2H), 3.71 (d, J = 2.4 Hz, 2H), 3.57 (t, J = 6.9 Hz, 2H), 2.34 (t, J = 7.5 Hz, 2H), 1.88 (p, J = 7.1 Hz, 2H).

13C NMR (101 MHz, DMSO) δ 174.88, 171.25, 171.11, 162.25, 159.88, 145.06, 144.94, 134.58, 132.44, 130.70, 130.29, 129.47, 127.92, 127.82, 125.77, 119.76, 102.35, 102.13, 101.75, 101.50, 38.20, 34.22, 33.27, 23.78.

### Example 7

### (E)-N-(2-amino-4-fluorophenyl)-5-(3-benzal-2,5-diketopyrrolidinyl)pentanamide (I-7)

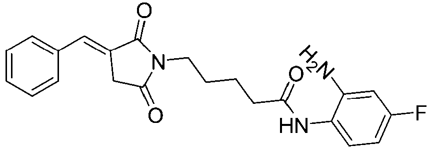

### Step a: Synthesis of (E)-3-benzalpyrrolidin-2,5-dione

The operation and feeding were the same as Step a in Example 1.

### Step b: Synthesis of (E)-5-(3-benzal-2,5-dioxopyrrolidinyl)ethyl pentanoate

The compound of interest was synthesized with a method similar to Step b in Example 1 by taking ethyl bromopentanoate as a starting material.

### Step c: Synthesis of (E)-N-(2-amino-4-fluorophenyl)-4-(3-benzal-2,5-diketopyrrolidinyl)butyramide

The compound of interest was synthesized with a method similar to Step c in Example 1 by taking ((E)-5-(3-benzal-2,5-dioxopyrrolidinyl)ethyl pentanoate and diamino-4-fluorobenzene as starting materials. The compound of interest was a white solid, with the yield of 36.8%. 1H NMR (300 MHz, DMSO-d6) δ 9.03 (s, 1H), 7.72 - 7.59 (m, 2H), 7.55 - 7.37 (m, 4H), 7.09 (dd, J = 8.7, 6.3 Hz, 1H), 6.48 (dd, J = 11.2, 2.9 Hz, 1H), 6.30 (td, J = 8.5, 2.9 Hz, 1H), 5.15 (s, 2H), 3.73 (d, J = 2.3 Hz, 2H), 3.53 (t, J = 5.5 Hz, 2H), 2.33 (t, J = 6.9 Hz, 2H), 1.65 - 1.50 (m, 4H).

13C NMR (101 MHz, DMSO) δ 174.82, 171.53, 171.19, 162.13, 159.77, 144.78, 144.66, 134.59, 132.42, 130.70, 130.28, 129.45, 127.62, 127.52, 125.77, 119.95, 119.92, 102.43, 102.21, 101.89, 101.64, 38.37, 35.55, 34.17, 27.42, 22.97.

### Example 8

### (E)-N-(2-amino-4-fluorophenyl)-6-(3-benzal-2,5-diketopyrrolidinyl)hexanamide (I-8)

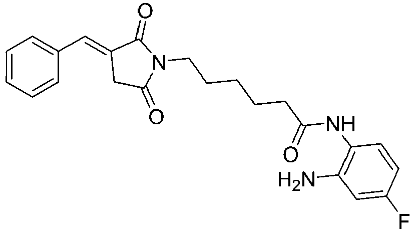

### Step a: Synthesis of (E)-3-benzalpyrrolidin-2,5-dione

The operation and feeding were the same as Step a in Example 1.

### Step b: Synthesis of (E)-6-(3-benzal-2,5-dioxopyrrolidinyl)ethyl hexanoate

The compound of interest was synthesized with a method similar to Step b in Example 1 by taking ethyl bromohexanoate as a starting material.

### Step c: Synthesis of (E)-N-(2-amino-4-fluorophenyl)-6-(3-benzal-2,5-diketopyrrolidinyl)hexanamide

The compound of interest was synthesized with a method similar to Step c in Example 1 by taking ((E)-6-(3-benzal-2,5-dioxopyrrolidinyl)ethyl hexanoate and diamino-4-fluorobenzene as starting materials. The compound of interest was a white solid, with the yield of 36.8%. 1H NMR (300 MHz, DMSO-d6) δ 9.03 (s, 1H), 7.72 - 7.59 (m, 2H), 7.55 - 7.37 (m, 4H), 7.09 (dd, J = 8.7, 6.3 Hz, 1H), 6.48 (dd, J = 11.2, 2.9 Hz, 1H), 6.30 (td, J = 8.5, 2.9 Hz, 1H), 5.15 (s, 2H), 3.73 (d, J = 2.3 Hz, 2H), 3.53 (t, J = 5.5 Hz, 2H), 2.33 (t, J = 6.9 Hz, 2H), 1.65 - 1.50 (m, 4H).

13C NMR (101 MHz, DMSO) δ 174.82, 171.53, 171.19, 162.13, 159.77, 144.78, 144.66, 134.59, 132.42, 130.70, 130.28, 129.45, 127.62, 127.52, 125.77, 119.95, 119.92, 102.43, 102.21, 101.89, 101.64, 38.37, 35.55, 34.17, 27.42, 22.97.

### Example 9

### (E)-N-(2-amino-4-fluorophenyl)-7-(3-benzal-2,5-diketopyrrolidinyl)heptamide (I-9)

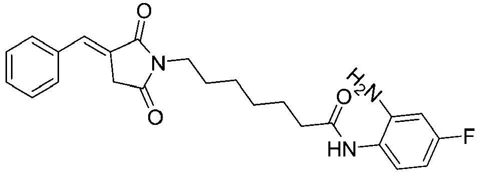

### Step a: Synthesis of (E)-3-benzalpyrrolidin-2,5-dione

The operation and feeding were the same as Step a in Example 1.

### Step b: Synthesis of (E)-7-(3-benzal-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-N-(2-amino-4-fluorophenyl)-7-(3-benzal-2,5-diketopyrrolidinyl)heptamide

The compound of interest was synthesized with a method similar to Step c in Example 1 by taking (E)-7-(3-benzal-2,5-dioxopyrrolidinyl)ethyl heptanoate and diamino-4-fluorobenzene as starting materials. The compound of interest was a white solid, with the yield of 32.9%. 1H NMR (300 MHz, DMSO-d6) δ 9.02 (s, 1H), 7.73 - 7.59 (m, 2H), 7.53 - 7.37 (m, 4H), 7.09 (dd, J = 8.7, 6.3 Hz, 1H), 6.48 (dd, J = 11.2, 2.9 Hz, 1H), 6.29 (td, J = 8.6, 2.9 Hz, 1H), 5.13 (s, 2H), 3.72 (d, J = 2.4 Hz, 2H), 3.50 (t, J = 7.1 Hz, 2H), 2.29 (t, J = 7.4 Hz, 2H), 1.67 - 1.47 (m, 4H), 1.40 - 1.23 (m, 4H).

13C NMR (101 MHz, DMSO) δ 174.81, 171.82, 171.18, 162.39, 159.61, 144.77, 144.65, 134.60, 132.38, 130.70, 130.27, 129.45, 127.59, 127.49, 125.77, 120.04, 102.47, 102.24, 101.93, 101.68, 38.49, 36.03, 34.15, 28.75, 27.69, 26.52, 25.56.

### Example 10

### 4-(3-((E)-benzal)-2,5-diketopyrrolidinyl)-N-((1S,2S)-2-hydroxycyclohexyl)butyramide (I-10)

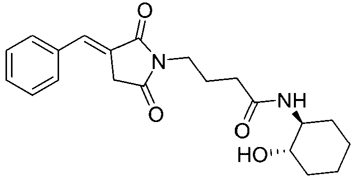

### Step a: Synthesis of (E)-3-benzalpyrrolidin-2,5-dione

The operation and feeding were the same as Step a in Example 1.

### Step b: Synthesis of (E)-4-(3-benzal-2,5-dioxopyrrolidinyl)ethyl butyrate

The compound of interest was synthesized with a method similar to Step b in Example 1 by taking ethyl bromobutyrate as a starting material.

### Step c: Synthesis of 4-(3-((E)-benzal)-2,5-diketopyrrolidinyl)-N-((1S,2S)-2-hydroxycyclohexyl)butyramide

The compound of interest was synthesized with a method similar to Step c in Example 1 by taking (E)-4-(3-benzal-2,5-dioxopyrrolidinyl)ethyl butyrate and (1S,2S)-2-aminocyclohexanol as starting materials. The compound of interest was a white solid, with the yield of 35.8%. 1H NMR (400 MHz, DMSO-d6) δ 7.68 - 7.63 (m, 2H), 7.59 (d, J = 8.0 Hz, 1H), 7.52 - 7.42 (m, 4H), 4.50 (d, J = 4.8 Hz, 1H), 3.70 (d, J = 2.4 Hz, 2H), 3.51 (t, J = 7.1 Hz, 3H), 3.41 (m, 1H), 3.20 (m, 1H), 2.10 (t, J = 7.0 Hz, 2H), 1.76 (p, J = 7.5 Hz, 2H), 1.64 - 1.51 (m, 2H), 1.31 - 0.99 (m, 6H).

13C NMR (101 MHz, DMSO) δ 174.80, 171.57, 171.18, 134.60, 132.38, 130.69, 130.27, 129.47, 125.79, 71.73, 54.58, 38.32, 34.40, 34.20, 33.46, 31.42, 24.54, 24.19, 24.13.

### Example 11

### 5-(3-((E)-benzal)-2,5-diketopyrrolidinyl)-N-((1S,2S)-2-hydroxycyclohexyl)pentanami de (I-11)

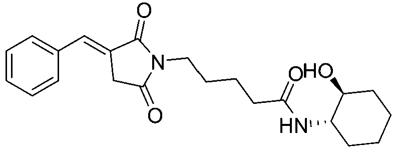

### Step a: Synthesis of (E)-3-benzalpyrrolidin-2,5-dione

The operation and feeding were the same as Step a in Example 1.

### Step b: Synthesis of (E)-5-(3-benzal-2,5-dioxopyrrolidinyl)ethyl pentanoate

The compound of interest was synthesized with a method similar to Step b in Example 1 by taking ethyl bromopentanoate as a starting material.

### Step c: Synthesis of 5-(3-((E)-benzal)-2,5-diketopyrrolidinyl)-N-((1S,2S)-2-hydroxycyclohexyl)pentanamide

The compound of interest was synthesized with a method similar to Step c in Example 1 by taking (E)-5-(3-benzal-2,5-dioxopyrrolidinyl)ethyl pentanoate and (1S,2S)-2-aminocyclohexanol as starting materials. The compound of interest was a white solid, with the yield of 40.6%. 1H NMR (400 MHz, DMSO-d6) δ 7.69 - 7.63 (m, 2H), 7.57 (d, J = 7.8 Hz, 1H), 7.51 - 7.42 (m, 4H), 4.50 (d, J = 4.9 Hz, 1H), 3.72 (d, J = 2.5 Hz, 2H), 3.49 (t, J = 6.5 Hz, 2H), 3.42 - 3.36 (m, 1H), 3.20 (m, 1H), 2.09 (t, J = 6.6 Hz, 2H), 1.87 - 1.72 (m, 2H), 1.62 - 1.43 (m, 6H), 1.24 - 1.06 (m, 4H).

13C NMR (101 MHz, DMSO) δ 174.78, 172.09, 171.15, 134.59, 132.41, 130.70, 130.27, 129.45, 125.76, 71.74, 54.54, 38.39, 35.50, 34.47, 34.15, 31.46, 27.43, 24.53, 24.20, 23.16.

### Example 12

### 6-(3-((E)-benzal)-2,5-diketopyrrolidinyl)-N-((1S,2S)-2-hydroxycyclohexyl)hexanamid e (I-12)

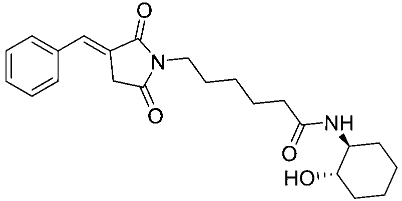

### Step a: Synthesis of (E)-3-benzalpyrrolidin-2,5-dione

The operation and feeding were the same as Step a in Example 1.

### Step b: Synthesis of (E)-6-(3-benzal-2,5-dioxopyrrolidinyl)ethyl hexanoate

The compound of interest was synthesized with a method similar to Step b in Example 1 by taking ethyl bromohexanoate as a starting material.

### Step c: Synthesis of 6-(3-((E)-benzal)-2,5-diketopyrrolidinyl)-N-((1S,2S)-2-hydroxycyclohexyl)hexanamide

The compound of interest was synthesized with a method similar to Step c in Example 1 by taking (E)-6-(3-benzal-2,5-dioxopyrrolidinyl)ethyl hexanoate and (1S,2S)-2-aminocyclohexanol as starting materials. The compound of interest was a white solid, with the yield of 38.5%. 1H NMR (400 MHz, DMSO-d6) δ 7.71 - 7.61 (m, 2H), 7.54 (d, J = 7.8 Hz, 1H), 7.52 - 7.41 (m, 4H), 4.48 (d, J = 5.1 Hz, 1H), 3.71 (d, J = 2.4 Hz, 2H), 3.48 (t, J = 7.1 Hz, 2H), 3.42 - 3.35 (m, 1H), 3.24 - 3.15 (m, 1H), 2.06 (t, J = 7.4 Hz, 2H), 1.86 - 1.70 (m, 2H), 1.64 - 1.45 (m, 6H), 1.30 - 1.00 (m, 6H).

13C NMR (101 MHz, DMSO) δ 174.75, 172.34, 171.13, 134.59, 132.39, 130.69, 130.26, 129.44, 125.74, 71.81, 54.55, 38.47, 35.81, 34.51, 34.14, 31.46, 27.61, 26.35, 25.41, 24.54, 24.21.

### Example 13

### 7-(3-((E)-benzal)-2,5-diketopyrrolidinyl)-N-((1S,2S)-2-hydroxycyclohexyl) heptamide (I-13)

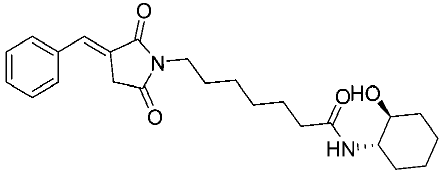

### Step a: Synthesis of (E)-3-benzalpyrrolidin-2,5-dione

The operation and feeding were the same as Step a in Example 1.

### Step b: Synthesis of (E)-7-(3-benzal-2,5-dioxopyrrolidinyl)ethyl heptanoate

The compound of interest was synthesized with a method similar to Step b in Example 1 by taking ethyl bromoheptanoate as a starting material.

### Step c: Synthesis of 7-(3-((E)-benzal)-2,5-diketopyrrolidinyl)-N-((1S,2S)-2-hydroxycyclohexyl) heptamide

The compound of interest was synthesized with a method similar to Step c in Example 1 by taking (E)-7-(3-benzal-2,5-dioxopyrrolidinyl)ethyl heptanoate and (1S,2S)-2-aminocyclohexanol as starting materials. The compound of interest was a white solid, with the yield of 48.1%. 1H NMR (400 MHz, DMSO-d6) δ 7.69 - 7.62 (m, 2H), 7.54 (d, J = 7.8 Hz, 1H), 7.51 - 7.42 (m, 4H), 4.48 (d, J = 5.0 Hz, 1H), 3.72 (d, J = 2.4 Hz, 2H), 3.48 (t, J = 7.2 Hz, 2H), 3.42 - 3.35 (m, 1H), 3.25 - 3.15 (m, 1H), 2.05 (t, J = 7.4 Hz, 2H), 1.87 - 1.70 (m, 2H), 1.64 - 1.40 (m, 6H), 1.29 - 1.22 (m, 4H), 1.22 - 1.07 (m, 4H).

13C NMR (101 MHz, DMSO) δ 174.79, 172.43, 171.16, 134.60, 132.37, 130.70, 130.26, 129.45, 125.78, 71.78, 54.53, 38.51, 35.94, 34.52, 34.14, 31.47, 28.70, 27.70, 26.53, 25.68, 24.54, 24.20.

### Example 14

### 8-(3-((E)-benzal)-2,5-diketopyrrolidinyl)-N-((1S,2S)-2-hydroxycyclohexyl)decoylami de (I-14)

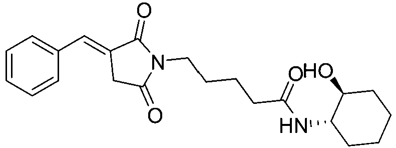

### Step a: Synthesis of (E)-3-benzalpyrrolidin-2,5-dione

The operation and feeding were the same as Step a in Example 1.

### Step b: Synthesis of (E)-8-(3-benzal-2,5-dioxopyrrolidinyl)ethyl octanoate

The compound of interest was synthesized with a method similar to Step b in Example 1 by taking ethyl bromooctanoate as a starting material.

### Step c: Synthesis of 8-(3-((E)-benzal)-2,5-diketopyrrolidinyl)-N-((1S,2S)-2-hydroxycyclohexyl)decoylamide

The compound of interest was synthesized with a method similar to Step c in Example 1 by taking (E)-8-(3-benzal-2,5-dioxopyrrolidinyl)ethyl octanoate and (1S,2S)-2-aminocyclohexanol as starting materials. The compound of interest was a white solid, with the yield of 36.2%. 1H NMR (400 MHz, DMSO-d6) δ 7.69 - 7.61 (m, 2H), 7.54 (d, J = 7.8 Hz, 1H), 7.51 - 7.43 (m, 4H), 4.49 (d, J = 5.0 Hz, 1H), 3.72 (d, J = 2.4 Hz, 2H), 3.48 (t, J = 7.2 Hz, 2H), 3.42 - 3.35 (m, 1H), 3.24 - 3.15 (m, 1H), 2.05 (t, J = 7.4 Hz, 2H), 1.87 - 1.71 (m, 2H), 1.61 - 1.42 (m, 6H), 1.31 - 1.09 (m, 10H).

13C NMR (101 MHz, DMSO) δ 174.77, 172.49, 171.15, 134.61, 132.38, 130.69, 130.25, 129.44, 125.77, 71.79, 54.54, 38.51, 36.02, 34.50, 34.14, 31.46, 28.97, 28.84, 27.75, 26.66, 25.75, 24.54, 24.20.

### Example 15

### (E)-7-(3-(4-isopropylbenzal)-2,5-diketopyrrolidinyl)-N-hydroxyheptamide (I-15)

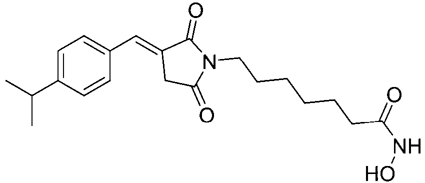

### Step a: Synthesis of (E)-3-(4-isopropylbenzal)pyrrolidin-2,5-dione

The compound of interest was synthesized with a method similar to Step a in Example 1 by taking 4-isopropylbenzaldehyde as a starting material.

### Step b: Synthesis of (E)-7-(3-(4-isopropylbenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-isopropylbenzal)-2,5-dioxopyrrolidinyl)-N-hydroxyheptamide

The compound of interest was synthesized with a method similar to Step c in Example 1 by taking ((E)-7-(3-(4-isopropylbenzal)-2,5-dioxopyrrolidinyl)ethyl heptanoate as a starting material. The compound of interest was a white solid, with the yield of 26.6%. 1H NMR (400 MHz, DMSO-d6) δ 10.33 (s, 1H), 8.65 (s, 1H), 7.57 (d, J = 8.1 Hz, 2H), 7.44 (t, J = 2.2 Hz, 1H), 7.36 (d, J = 8.2 Hz, 2H), 3.70 (d, J = 2.4 Hz, 2H), 3.47 (t, J = 7.2 Hz, 2H), 2.94 (hept, J = 6.8 Hz, 1H), 1.93 (t, J = 7.3 Hz, 2H), 1.56 - 1.42 (m, 4H), 1.28 - 1.20 (m, 10H).

13C NMR (101 MHz, DMSO) δ 174.84, 171.25, 169.51, 150.98, 132.40, 132.29, 130.87, 127.44, 124.69, 38.46, 34.14, 33.82, 32.64, 28.63, 27.68, 26.45, 25.45, 24.08.

### Example 16

### (E)-7-(3-(4-dimethylaminobenzal)-2,5-diketopyrrolidinyl)-N-hydroxyheptamide (I-16)

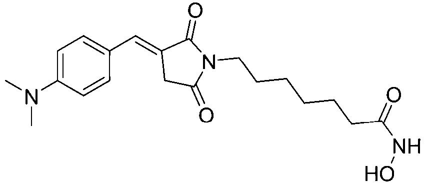

### Step a: Synthesis of (E)-3-(4-dimethylaminobenzal)pyrrolidin-2,5-dione

The compound of interest was synthesized with a method similar to Step a in Example 1 by taking 4-dimethylaminobenzaldehyde as a starting material.

### Step b: Synthesis of (E)-7-(3-(4-dimethylaminobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-dimethylaminobenzal)-2,5-dioxopyrrolidinyl)-N-hydroxyheptamide

The compound of interest was synthesized with a method similar to Step c in Example 1 by taking ((E)-7-(3-(4-dimethylaminobenzal)-2,5-dioxopyrrolidinyl)ethyl heptanoate as a starting material. The compound of interest was a yellow solid, with the yield of 32.1%. 1H NMR (300 MHz, DMSO-d6) δ 10.35 (s, 1H), 8.68 (s, 1H), 7.46 (d, J = 8.5 Hz, 2H), 7.35 (t, J = 2.0 Hz, 1H), 6.76 (d, J = 8.4 Hz, 2H), 3.61 (d, J = 2.2 Hz, 2H), 3.45 (t, J = 7.1 Hz, 2H), 2.99 (s, 6H), 1.93 (t, J = 7.3 Hz, 2H), 1.57 - 1.41 (m, 4H), 1.30 - 1.15 (m, 4H).

13C NMR (101 MHz, DMSO) δ 174.98, 171.51, 169.53, 151.56, 133.35, 132.48, 121.95, 119.01, 112.34, 40.10, 38.29, 34.25, 32.65, 28.64, 27.77, 26.46, 25.46.

### Example 17

### (E)-7-(3-(4-trifluoromethylbenzal)-2,5-diketopyrrolidinyl)-N-hydroxyheptamide (I-17)

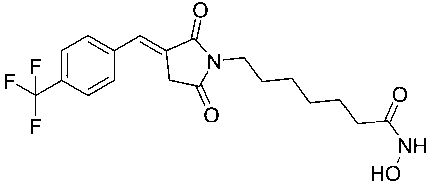

### Step a: Synthesis of (E)-3-(4-trifluoromethylbenzal)pyrrolidin-2,5-dione

The compound of interest was synthesized with a method similar to Step a in Example 1 by taking 4-trifluoromethylbenzaldehyde as a starting material.

### Step b: Synthesis of (E)-7-(3-(4-trifluoromethylbenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-trifluoromethylbenzal)-2,5-dioxopyrrolidinyl)-N-hydroxyheptamide

The compound of interest was synthesized with a method similar to Step c in Example 1 by taking ((E)-7-(3-(4-trifluoromethylbenzal)-2,5-dioxopyrrolidinyl)ethyl heptanoate as a starting material. The compound of interest was a white solid, with the yield of 15.8%. 1H NMR (400 MHz, DMSO-d6) δ 10.33 (s, 1H), 8.65 (s, 1H), 7.88 (d, J = 8.3 Hz, 2H), 7.82 (d, J = 8.3 Hz, 2H), 7.55 (t, J = 2.3 Hz, 1H), 3.77 (d, J = 2.4 Hz, 2H), 3.49 (t, J = 7.2 Hz, 2H), 1.93 (t, J = 7.4 Hz, 2H), 1.59 - 1.43 (m, 4H), 1.29 - 1.22 (m, 4H).

13C NMR (101 MHz, DMSO) δ 174.60, 170.84, 169.51, 138.61, 131.19, 130.53, 129.91, 129.59, 128.77, 126.16, 126.12, 125.83, 123.12, 38.58, 34.13, 32.63, 28.62, 27.62, 26.43, 25.44.

### Example 18

### (E)-7-(3-(4-methylbenzal)-2,5-diketopyrrolidinyl)-N-hydroxyheptamide (I-18)

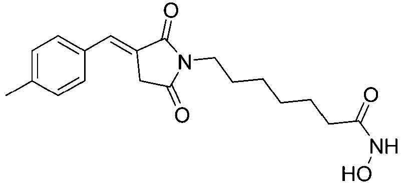

### Step a: Synthesis of (E)-3-(4-methylbenzal)pyrrolidin-2,5-dione

The compound of interest was synthesized with a method similar to Step a in Example 1 by taking 4-methylbenzaldehyde as a starting material.

### Step b: Synthesis of (E)-7-(3-(4-methylbenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-methylbenzal)-2,5-dioxopyrrolidinyl)-N-hydroxyheptamide

The compound of interest was synthesized with a method similar to Step c in Example 1 by taking ((E)-7-(3-(4-methylbenzal)-2,5-dioxopyrrolidinyl)ethyl heptanoate as a starting material. The compound of interest was a white solid, with the yield of 26.7%. 1H NMR (400 MHz, DMSO-d6) δ 10.33 (s, 1H), 8.65 (s, 1H), 7.54 (d, J = 5.8 Hz, 2H), 7.47 - 7.40 (m, 1H), 7.30 (d, J = 5.8 Hz, 2H), 3.69 (d, J = 2.9 Hz, 2H), 3.48 (t, J = 6.0 Hz, 2H), 2.36 (s, 3H), 1.94 (t, J = 6.9 Hz, 2H), 1.56 - 1.41 (m, 4H), 1.30 - 1.19 (m, 4H).

13C NMR (101 MHz, DMSO) δ 174.82, 171.24, 169.52, 140.28, 132.42, 131.88, 130.73, 130.08, 124.62, 38.44, 34.16, 32.64, 28.62, 27.68, 26.43, 25.44, 21.48.

### Example 19

### (E)-7-(3-(2-methylbenzal)-2,5-diketopyrrolidinyl)-N-hydroxyheptamide (I-19)

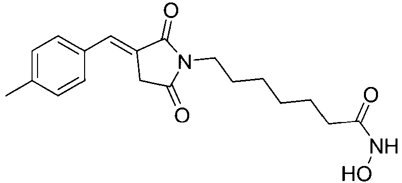

### Step a: Synthesis of (E)-3-(2-methylbenzal)pyrrolidin-2,5-dione

The compound of interest was synthesized with a method similar to Step a in Example 1 by taking 2-methylbenzaldehyde as a starting material.

### Step b: Synthesis of (E)-7-(3-(2-methylbenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-methylbenzal)-2,5-dioxopyrrolidinyl)-N-hydroxyheptamide

The compound of interest was synthesized with a method similar to Step c in Example 1 by taking ((E)-7-(3-(2-methylbenzal)-2,5-dioxopyrrolidinyl)ethyl heptanoate as a starting material. The compound of interest was a white solid, with the yield of 35.2%. 1H NMR (400 MHz, DMSO-d6) δ 10.35 (s, 1H), 8.68 (s, 1H), 7.64 (t, J = 2.4 Hz, 1H), 7.56 (d, J = 7.1 Hz, 1H), 7.36 - 7.25 (m, 3H), 3.65 (d, J = 2.4 Hz, 2H), 3.48 (t, J = 7.2 Hz, 2H), 2.39 (s, 3H), 1.93 (t, J = 7.4 Hz, 2H), 1.58 - 1.44 (m, 4H), 1.29 - 1.20 (m, 4H).

13C NMR (101 MHz, DMSO) δ 174.88, 171.07, 169.57, 138.63, 133.24, 131.10, 130.06, 129.81, 128.65, 126.81, 126.45, 38.47, 33.77, 32.64, 28.61, 27.66, 26.44, 25.43, 19.94.

### Example 20

### (E)-7-(3-(2-cyanobenzal)-2,5-diketopyrrolidinyl)-N-hydroxyheptamide (I-20)

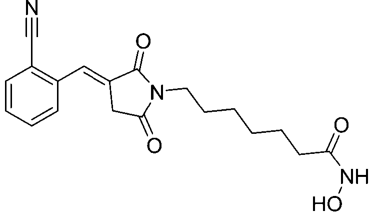

### Step a: Synthesis of (E)-3-(2-cyanobenzal)pyrrolidin-2,5-dione

The compound of interest was synthesized with a method similar to Step a in Example 1 by taking 2-cyanobenzaldehyde as a starting material.

### Step b: Synthesis of (E)-7-(3-(2-cyanobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-cyanobenzal)-2,5-dioxopyrrolidinyl)-N-hydroxyheptamide

The compound of interest was synthesized with a method similar to Step c in Example 1 by taking ((E)-7-(3-(2-cyanobenzal)-2,5-dioxopyrrolidinyl)ethyl heptanoate as a starting material. The compound of interest was a white solid, with the yield of 12.1%. 1H NMR (400 MHz, DMSO-d6) δ 10.33 (s, 1H), 8.65 (s, 1H), 7.99 (dd, J = 7.8, 1.4 Hz, 1H), 7.90 (d, J = 7.9 Hz, 1H), 7.82 (td, J = 7.8, 1.4 Hz, 1H), 7.67 - 7.61 (m, 2H), 3.77 (d, J = 2.5 Hz, 2H), 3.50 (t, J = 7.1 Hz, 2H), 1.94 (t, J = 7.4 Hz, 2H), 1.60 - 1.44 (m, 4H), 1.30 - 1.22 (m, 4H).

13C NMR (101 MHz, DMSO) δ 174.43, 170.56, 169.53, 136.91, 134.17, 134.16, 130.86, 130.59, 129.42, 126.63, 117.72, 113.25, 38.70, 34.04, 32.65, 28.62, 27.59, 26.44, 25.44.

### Example 21

### (E)-7-(3-(2-methylenepyridin)-2,5-diketopyrrolidinyl)-N-hydroxyheptamide (I-21)

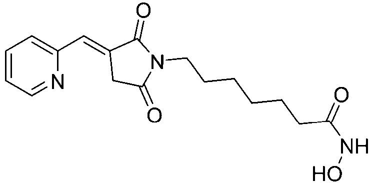

### Step a: Synthesis of (E)-3-(2-methylenepyridin)pyrrolidin-2,5-dione

The compound of interest was synthesized with a method similar to Step a in Example 1 by taking 2-pyridylaldehyde as a starting material.

### Step b: Synthesis of (E)-7-(3-(2-methylenepyridin)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-methylenepyridin)-2,5-dioxopyrrolidinyl)-N-hydroxyheptamide

The compound of interest was synthesized with a method similar to Step c in Example 1 by taking ((E)-7-(3-(2-methylenepyridin)-2,5-dioxopyrrolidinyl)ethyl heptanoate as a starting material. The compound of interest was a yellow solid, with the yield of 16.8%. 1H NMR (300 MHz, DMSO-d6) δ 10.35 (s, 1H), 8.73 (d, J = 4.5 Hz, 1H), 8.68 (s, 1H), 7.90 (td, J = 7.7, 1.8 Hz, 1H), 7.76 (d, J = 7.7 Hz, 1H), 7.48 (t, J = 2.2 Hz, 1H), 7.40 (ddd, J = 7.5, 4.7, 1.3 Hz, 1H), 3.78 (d, J = 2.3 Hz, 2H), 3.48 (t, J = 7.2 Hz, 2H), 1.93 (t, J = 7.3 Hz, 2H), 1.59 - 1.42 (m, 4H), 1.32 - 1.19 (m, 4H).

13C NMR (101 MHz, DMSO) δ 175.36, 171.10, 169.66, 153.68, 150.53, 137.58, 130.42, 129.55, 127.54, 124.19, 38.45, 35.37, 32.61, 28.58, 27.62, 26.40, 25.41.

### Example 22

### (E)-7-(3-(2-methylenenaphthyl)-2,5-diketopyrrolidinyl)-N-hydroxyheptamide (I-22)

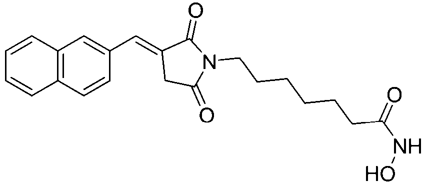

### Step a: Synthesis of (E)-3-(2-methylenenaphthyl)pyrrolidin-2,5-dione

The compound of interest was synthesized with a method similar to Step a in Example 1 by taking 2-naphthaldehyde as a starting material.

### Step b: Synthesis of (E)-7-(3-(2-methylenenaphthyl)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-methylenenaphthyl)-2,5-dioxopyrrolidinyl)-N-hydroxyheptamide

The compound of interest was synthesized with a method similar to Step c in Example 1 by taking ((E)-7-(3-(2-methylenenaphthyl)-2,5-dioxopyrrolidinyl)ethyl heptanoate as a starting material. The compound of interest was a white solid, with the yield of 18.2%. 1H NMR (400 MHz, DMSO-d6) δ 10.33 (s, 1H), 8.66 (s, 1H), 8.25 (s, 1H), 8.07 - 7.92 (m, 3H), 7.77 (dd, J = 8.6, 1.8 Hz, 1H), 7.63 (t, J = 2.4 Hz, 1H), 7.61 - 7.55 (m, 2H), 3.85 (d, J = 2.3 Hz, 2H), 3.50 (t, J = 7.2 Hz, 2H), 1.94 (t, J = 7.4 Hz, 2H), 1.50 (m, 4H), 1.27 (m, 4H).

13C NMR (101 MHz, DMSO) δ 174.88, 171.20, 169.56, 133.59, 133.33, 132.41, 132.20, 131.18, 129.11, 128.94, 128.05, 127.97, 127.23, 127.18, 126.05, 38.52, 34.29, 32.64, 28.62, 27.67, 26.45, 25.45.

### Example 23

### (E)-7-(3-(2-methylenethienyl)-2,5-diketopyrrolidinyl)-N-hydroxyheptamide (I-23)

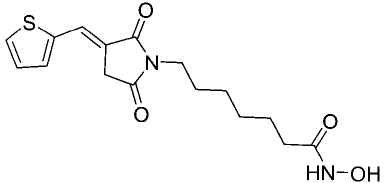

### Step a: Synthesis of (E)-3-(2-methylenethienyl)pyrrolidin-2,5-dione

The compound of interest was synthesized with a method similar to Step a in Example 1 by taking 2-thiophenecarboxaldehyde as a starting material.

### Step b: Synthesis of (E)-7-(3-(2-methylenethienyl)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-methylenethienyl)-2,5-dioxopyrrolidinyl)-N-hydroxyheptamide

The compound of interest was synthesized with a method similar to Step c in Example 1 by taking ((E)-7-(3-(2-methylenethienyl)-2,5-dioxopyrrolidinyl)ethyl heptanoate as a starting material. The compound of interest was a yellow solid, with the yield of 18.6%. 1H NMR (400 MHz, DMSO-d6) δ 10.36 (s, 1H), 8.69 (s, 1H), 7.90 (d, J = 5.0 Hz, 1H), 7.72 (t, J = 2.4 Hz, 1H), 7.60 (d, J = 3.7 Hz, 1H), 7.24 (t, J = 4.3 Hz, 1H), 3.52 (d, J = 2.3 Hz, 2H), 3.46 (t, J = 7.2 Hz, 2H), 1.93 (t, J = 7.3 Hz, 2H), 1.58 - 1.42 (m, 4H), 1.29 - 1.18 (m, 4H).

13C NMR (101 MHz, DMSO) δ 174.23, 170.77, 169.55, 138.65, 133.73, 132.12, 128.88, 125.49, 122.97, 38.52, 34.12, 32.63, 28.62, 27.69, 26.42, 25.45.

### Example 24

### (E)-7-(3-(2-chlorobenzal)-2,5-diketopyrrolidinyl)-N-hydroxyheptamide (I-24)

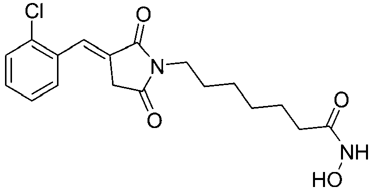

### Step a: Synthesis of (E)-3-(2-chlorobenzal)pyrrolidin-2,5-dione

The compound of interest was synthesized with a method similar to Step a in Example 1 by taking 2-chlorobenzaldehyde as a starting material.

### Step b: Synthesis of (E)-7-(3-(2-chlorobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-chlorobenzal)-2,5-dioxopyrrolidinyl)-N-hydroxyheptamide

The compound of interest was synthesized with a method similar to Step c in Example 1 by taking ((E)-7-(3-(2-chlorobenzal)-2,5-dioxopyrrolidinyl)ethyl heptanoate as a starting material. The compound of interest was a faint yellow solid, with the yield of 10.2%. 1H NMR (300 MHz, DMSO-d6) δ 10.34 (s, 1H), 8.67 (s, 1H), 7.80 - 7.73 (m, 1H), 7.71 (t, J = 2.2 Hz, 1H), 7.64 - 7.57 (m, 1H), 7.50 - 7.41 (m, 2H), 3.71 (d, J = 2.4 Hz, 2H), 3.49 (t, J = 7.1 Hz, 2H), 1.94 (t, J = 7.3 Hz, 2H), 1.60 - 1.40 (m, 4H), 1.31 - 1.18 (m, 4H).

13C NMR (101 MHz, DMSO) δ 174.63, 170.79, 169.55, 134.82, 132.19, 131.72, 130.45, 130.42, 128.77, 128.23, 127.33, 38.59, 33.68, 32.64, 28.61, 27.61, 26.43, 25.43.

### Example 25

### (E)-7-(3-(3-chlorobenzal)-2,5-diketopyrrolidinyl)-N-hydroxyheptamide (I-25)

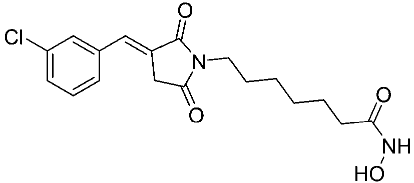

### Step a: Synthesis of (E)-3-(3-chlorobenzal)pyrrolidin-2,5-dione

The compound of interest was synthesized with a method similar to Step a in Example 1 by taking 3-chlorobenzaldehyde as a starting material.

### Step b: Synthesis of (E)-7-(3-(3-chlorobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3-chlorobenzal)-2,5-dioxopyrrolidinyl)-N-hydroxyheptamide

The compound of interest was synthesized with a method similar to Step c in Example 1 by taking ((E)-7-(3-(3-chlorobenzal)-2,5-dioxopyrrolidinyl)ethyl heptanoate as a starting material. The compound of interest was a faint yellow solid, with the yield of 12.6%. 1H NMR (400 MHz, DMSO-d6) δ 10.33 (s, 1H), 8.65 (s, 1H), 7.72 (s, 1H), 7.65 - 7.60 (m, 1H), 7.50 (d, J = 4.5 Hz, 2H), 7.47 (t, J = 2.4 Hz, 1H), 3.75 (d, J = 2.4 Hz, 2H), 3.48 (t, J = 7.2 Hz, 2H), 1.93 (t, J = 7.3 Hz, 2H), 1.57 - 1.42 (m, 4H), 1.30 - 1.21 (m, 4H).

13C NMR (101 MHz, DMSO) δ 174.67, 170.93, 169.59, 136.74, 134.14, 131.23, 130.83, 130.18, 129.93, 128.97, 127.49, 38.54, 34.00, 32.62, 28.59, 27.61, 26.41, 25.43.

### Example 26

### (E)-7-(3-(4-chlorobenzal)-2,5-diketopyrrolidinyl)-N-hydroxyheptamide (I-26)

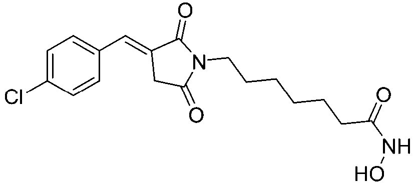

### Step a: Synthesis of (E)-3-(4-chlorobenzal)pyrrolidin-2,5-dione

The compound of interest was synthesized with a method similar to Step a in Example 1 by taking 4-chlorobenzaldehyde as a starting material.

### Step b: Synthesis of (E)-7-(3-(4-chlorobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-chlorobenzal)-2,5-dioxopyrrolidinyl)-N-hydroxyheptamide

The compound of interest was synthesized with a method similar to Step c in Example 1 by taking ((E)-7-(3-(4-chlorobenzal)-2,5-dioxopyrrolidinyl)ethyl heptanoate as a starting material. The compound of interest was a yellow solid, with the yield of 15.8%. 1H NMR (400 MHz, DMSO-d6) δ 10.33 (s, 1H), 8.65 (s, 1H), 7.68 (d, J = 8.7 Hz, 2H), 7.54 (d, J = 8.5 Hz, 2H), 7.47 (t, J = 2.4 Hz, 1H), 3.71 (d, J = 2.4 Hz, 2H), 3.48 (t, J = 7.2 Hz, 2H), 1.93 (t, J = 7.4 Hz, 2H), 1.59 - 1.42 (m, 4H), 1.33 - 1.20 (m, 4H).

13C NMR (101 MHz, DMSO) δ 174.67, 171.01, 169.55, 134.86, 133.53, 132.35, 131.01, 129.46, 126.59, 38.52, 34.07, 32.64, 28.61, 27.63, 26.42, 25.43.

### Example 27

### (E)-7-(3-(3-bromobenzal)-2,5-diketopyrrolidinyl)-N-hydroxyheptamide (I-27)

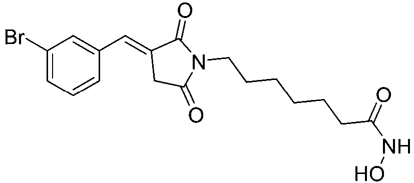

### Step a: Synthesis of (E)-3-(3-bromobenzal)pyrrolidin-2,5-dione

The compound of interest was synthesized with a method similar to Step a in Example 1 by taking 3-bromobenzaldehyde as a starting material.

### Step b: Synthesis of (E)-7-(3-(3-bromobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3-bromobenzal)-2,5-dioxopyrrolidinyl)-N-hydroxyheptamide

The compound of interest was synthesized with a method similar to Step c in Example 1 by taking ((E)-7-(3-(3-bromobenzal)-2,5-dioxopyrrolidinyl)ethyl heptanoate as a starting material. The compound of interest was a white solid, with the yield of 26.2%. 1H NMR (400 MHz, DMSO-d6) δ 10.35 (s, 1H), 8.68 (s, 1H), 7.85 (s, 1H), 7.70 - 7.60 (m, 2H), 7.46 (t, J = 2.3 Hz, 1H), 7.43 (d, J = 7.8 Hz, 1H), 3.75 (d, J = 2.4 Hz, 2H), 3.48 (t, J = 7.1 Hz, 2H), 1.93 (t, J = 7.3 Hz, 2H), 1.57 - 1.43 (m, 4H), 1.29 - 1.21 (m, 4H).

13C NMR (101 MHz, DMSO) δ 174.67, 170.90, 169.52, 137.03, 133.11, 132.82, 131.47, 130.75, 129.27, 127.49, 122.71, 38.53, 34.00, 32.63, 28.61, 27.64, 26.43, 25.44.

### Example 28

### (E)-7-(3-(3-fluorobenzal)-2,5-diketopyrrolidinyl)-N-hydroxyheptamide (I-28)

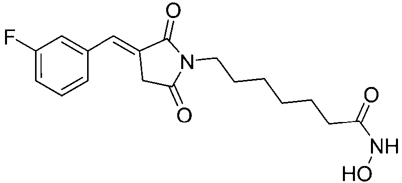

### Step a: Synthesis of (E)-3-(3-fluorobenzal)pyrrolidin-2,5-dione

The compound of interest was synthesized with a method similar to Step a in Example 1 by taking 3-fluorobenzaldehyde as a starting material.

### Step b: Synthesis of (E)-7-(3-(3-fluorobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3-fluorobenzal)-2,5-dioxopyrrolidinyl)-N-hydroxyheptamide

The compound of interest was synthesized with a method similar to Step c in Example 1 by taking ((E)-7-(3-(3-fluorobenzal)-2,5-dioxopyrrolidinyl)ethyl heptanoate as a starting material. The compound of interest was a white solid, with the yield of 29.6%. 1H NMR (400 MHz, DMSO-d6) δ 10.33 (s, 1H), 8.66 (s, 1H), 7.59 - 7.44 (m, 4H), 7.34 - 7.22 (m, 1H), 3.75 (d, J = 2.4 Hz, 2H), 3.48 (t, J = 7.2 Hz, 2H), 1.93 (t, J = 7.4 Hz, 2H), 1.58 - 1.42 (m, 4H), 1.32 - 1.18 (m, 4H).

13C NMR (101 MHz, DMSO) δ 174.69, 170.96, 169.52, 163.94, 161.51, 136.99, 136.91, 131.42, 131.34, 131.03, 127.35, 126.85, 126.82, 117.13, 117.06, 116.92, 116.84, 38.53, 34.02, 32.63, 28.62, 27.64, 26.43, 25.44.

### Example 29

### (E)-7-(3-(3-iodobenzal)-2,5-diketopyrrolidinyl)-N-hydroxyheptamide (I-29)

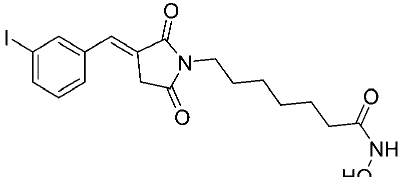

### Step a: Synthesis of (E)-3-(3-iodobenzal)pyrrolidin-2,5-dione

The compound of interest was synthesized with a method similar to Step a in Example 1 by taking 3-iodobenzaldehyde as a starting material.

### Step b: Synthesis of (E)-7-(3-(3-iodobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3-iodobenzal)-2,5-dioxopyrrolidinyl)-N-hydroxyheptamide

The compound of interest was synthesized with a method similar to Step c in Example 1 by taking ((E)-7-(3-(3-iodobenzal)-2,5-dioxopyrrolidinyl)ethyl heptanoate as a starting material. The compound of interest was a white solid, with the yield of 18.6%. 1H NMR (300 MHz, DMSO-d6) δ 10.35 (s, 1H), 8.69 (s, 1H), 8.00 (s, 1H), 7.80 (d, J = 7.6 Hz, 1H), 7.67 (d, J = 7.8 Hz, 1H), 7.41 (t, J = 2.4 Hz, 1H), 7.27 (t, J = 7.8 Hz, 1H), 3.73 (d, J = 2.4 Hz, 2H), 3.47 (t, J = 7.1 Hz, 2H), 1.93 (t, J = 7.3 Hz, 2H), 1.57 - 1.40 (m, 4H), 1.29 - 1.18 (m, 4H).

13C NMR (101 MHz, DMSO) δ 174.68, 170.91, 169.52, 138.95, 138.67, 136.92, 131.41, 130.79, 129.55, 127.19, 95.92, 38.52, 33.98, 32.62, 28.61, 27.64, 26.42, 25.44.

### Example 30

### (E)-7-(3-(3,4-dichlorobenzal)-2,5-diketopyrrolidinyl)-N-hydroxyheptamide (I-30)

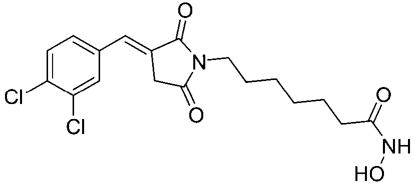

### Step a: Synthesis of (E)-3-(3,4-dichlorobenzal)pyrrolidin-2,5-dione

The compound of interest was synthesized with a method similar to Step a in Example 1 by taking 3,4-dichlorobenzaldehyde as a starting material.

### Step b: Synthesis of (E)-7-(3-(3,4-dichlorobenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3,4-dichlorobenzal)-2,5-dioxopyrrolidinyl)-N-hydroxyheptamide

The compound of interest was synthesized with a method similar to Step c in Example 1 by taking ((E)-7-(3-(3,4-dichlorobenzal)-2,5-dioxopyrrolidinyl)ethyl heptanoate as a starting material. The compound of interest was a yellow solid, with the yield of 18.5%. 1H NMR (400 MHz, DMSO-d6) δ 10.33 (s, 1H), 8.65 (s, 1H), 7.92 (s, 1H), 7.72 (d, J = 8.3 Hz, 1H), 7.64 (d, J = 8.4 Hz, 1H), 7.46 (s, 1H), 3.75 (s, 2H), 3.48 (t, J = 7.1 Hz, 2H), 1.93 (t, J = 7.3 Hz, 2H), 1.57 - 1.41 (m, 4H), 1.30 - 1.17 (m, 4H).

13C NMR (101 MHz, DMSO) δ 174.56, 170.78, 169.51, 135.38, 132.63, 132.33, 132.19, 131.48, 130.26, 129.78, 128.08, 38.57, 33.94, 32.64, 28.62, 27.62, 26.43, 25.44.

### Example 31

### (E)-7-(3-(2-methoxybenzal)-2,5-diketopyrrolidinyl)-N-hydroxyheptamide (I-31)

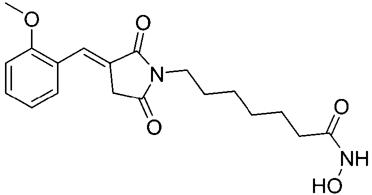

### Step a: Synthesis of (E)-3-(2-methoxybenzal)pyrrolidin-2,5-dione

The compound of interest was synthesized with a method similar to Step a in Example 1 by taking 2-methoxybenzaldehyde as a starting material.

### Step b: Synthesis of (E)-7-(3-(2-methoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-methoxybenzal)-2,5-dioxopyrrolidinyl)-N-hydroxyheptamide

The compound of interest was synthesized with a method similar to Step c in Example 1 by taking ((E)-7-(3-(2-methoxybenzal)-2,5-dioxopyrrolidinyl)ethyl heptanoate as a starting material. The compound of interest was a white solid, with the yield of 19.3%. 1H NMR (400 MHz, DMSO-d6) δ 10.33 (s, 1H), 8.65 (s, 1H), 7.77 (t, J = 2.4 Hz, 1H), 7.57 (dd, J = 7.8, 1.6 Hz, 1H), 7.44 (ddd, J = 8.7, 7.3, 1.7 Hz, 1H), 7.12 (dd, J = 8.4, 1.1 Hz, 1H), 7.07 - 7.01 (m, 1H), 3.88 (s, 3H), 3.66 (d, J = 2.4 Hz, 2H), 3.47 (t, J = 7.1 Hz, 2H), 1.93 (t, J = 7.4 Hz, 2H), 1.56 - 1.42 (m, 4H), 1.29 - 1.20 (m, 4H).

13C NMR (101 MHz, DMSO) δ 174.92, 171.26, 169.57, 158.48, 132.06, 129.58, 126.54, 125.25, 122.92, 121.16, 111.99, 56.18, 38.43, 33.95, 32.63, 28.61, 27.67, 26.43, 25.44.

### Example 32

### (E)-7-(3-(3-methoxybenzal)-2,5-diketopyrrolidinyl)-N-hydroxyheptamide (I-32)

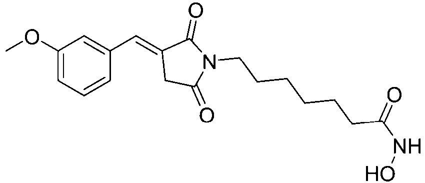

### Step a: Synthesis of (E)-3-(3-methoxybenzal)pyrrolidin-2,5-dione

The compound of interest was synthesized with a method similar to Step a in Example 1 by taking 3-methoxybenzaldehyde as a starting material.

### Step b: Synthesis of (E)-7-(3-(3-methoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3-methoxybenzal)-2,5-dioxopyrrolidinyl)-N-hydroxyheptamide

The compound of interest was synthesized with a method similar to Step c in Example 1 by taking ((E)-7-(3-(3-methoxybenzal)-2,5-dioxopyrrolidinyl)ethyl heptanoate as a starting material. The compound of interest was a white solid, with the yield of 10.3%. 1H NMR (400 MHz, DMSO-d6) δ 10.35 (s, 1H), 8.68 (s, 1H), 7.45 (s, 1H), 7.39 (t, J = 8.0 Hz, 1H), 7.22 (d, J = 7.8 Hz, 1H), 7.19 (s, 1H), 7.02 (d, J = 8.2 Hz, 1H), 3.81 (s, 3H), 3.74 (s, 2H), 3.48 (t, J = 7.2 Hz, 2H), 1.93 (t, J = 7.4 Hz, 2H), 1.61 - 1.41 (m, 4H), 1.32 - 1.20 (m, 4H).

13C NMR (101 MHz, DMSO) δ 174.77, 171.12, 169.52, 159.94, 135.93, 132.39, 130.49, 126.01, 122.96, 116.23, 115.69, 55.67, 38.48, 34.08, 32.64, 28.62, 27.66, 26.43, 25.45.

### Example 33

### (E)-7-(3-(4-methoxybenzal)-2,5-diketopyrrolidinyl)-N-hydroxyheptamide (I-33)

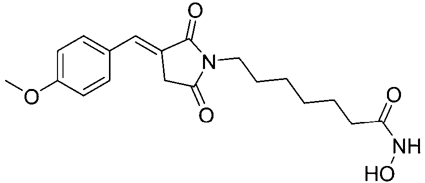

### Step a: Synthesis of (E)-3-(4-methoxybenzal)pyrrolidin-2,5-dione

The compound of interest was synthesized with a method similar to Step a in Example 1 by taking 4-methoxybenzaldehyde as a starting material.

### Step b: Synthesis of (E)-7-(3-(4-methoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-methoxybenzal)-2,5-dioxopyrrolidinyl)-N-hydroxyheptamide

The compound of interest was synthesized with a method similar to Step c in Example 1 by taking ((E)-7-(3-(4-methoxybenzal)-2,5-dioxopyrrolidinyl)ethyl heptanoate as a starting material. The compound of interest was a white solid, with the yield of 12.5%. 1H NMR (400 MHz, DMSO-d6) δ 10.34 (s, 1H), 8.67 (s, 1H), 7.61 (d, J = 9.1 Hz, 2H), 7.43 (t, J = 2.4 Hz, 1H), 7.04 (d, J = 7.9 Hz, 2H), 3.82 (s, 3H), 3.66 (d, J = 2.1 Hz, 2H), 3.46 (t, J = 7.2 Hz, 2H), 1.93 (t, J = 7.3 Hz, 2H), 1.53 - 1.43 (m, 4H), 1.26 - 1.22 (m, 4H).

13C NMR (101 MHz, DMSO) δ 174.89, 171.35, 169.58, 160.98, 132.60, 132.32, 127.21, 122.77, 114.97, 55.82, 38.40, 34.08, 32.63, 28.61, 27.69, 26.43, 25.44.

### Example 34

### (E)-7-(3-(3,4-dimethoxybenzal)-2,5-diketopyrrolidinyl)-N-hydroxyheptamide (I-34)

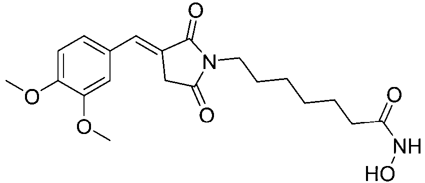

### Step a: Synthesis of (E)-3-(3,4-dimethoxybenzal)pyrrolidin-2,5-dione

The compound of interest was synthesized with a method similar to Step a in Example 1 by taking 3,4-dimethoxybenzaldehyde as a starting material.

### Step b: Synthesis of (E)-7-(3-(3,4-dimethoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3,4-dimethoxybenzal)-2,5-dioxopyrrolidinyl)-N-hydroxyheptamide

The compound of interest was synthesized with a method similar to Step c in Example 1 by taking ((E)-7-(3-(3,4-dimethoxybenzal)-2,5-dioxopyrrolidinyl)ethyl heptanoate as a starting material. The compound of interest was a white solid, with the yield of 21.5%. 1H NMR (400 MHz, DMSO-d6) δ 10.33 (s, 1H), 8.65 (s, 1H), 7.43 (t, J = 2.3 Hz, 1H), 7.23 (dd, J = 8.4, 2.1 Hz, 1H), 7.20 (d, J = 2.0 Hz, 1H), 7.05 (d, J = 8.4 Hz, 1H), 3.83 (s, 3H), 3.82 (s, 3H), 3.73 (d, J = 2.3 Hz, 2H), 3.47 (t, J = 7.1 Hz, 2H), 1.93 (t, J = 7.3 Hz, 2H), 1.54 - 1.43 (m, 4H), 1.28 - 1.22 (m, 4H).

13C NMR (101 MHz, DMSO) δ 174.92, 171.32, 169.52, 150.82, 149.22, 132.76, 127.38, 124.63, 122.83, 113.58, 112.26, 56.06, 55.99, 38.41, 33.98, 32.64, 28.63, 27.70, 26.44, 25.46.

### Example 35

### (E)-7-(3-(3,4,5-trimethoxybenzal)-2,5-diketopyrrolidinyl)-N-hydroxyheptamide (I-35)

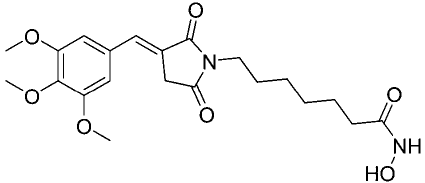

### Step a: Synthesis of (E)-3-(3,4,5-trimethoxybenzal)pyrrolidin-2,5-dione

The compound of interest was synthesized with a method similar to Step a in Example 1 by taking 3,4,5-trimethoxybenzaldehyde as a starting material.

### Step b: Synthesis of (E)-7-(3-(3,4,5-trimethoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(3,4,5-trimethoxybenzal)-2,5-dioxopyrrolidinyl)-N-hydroxyheptamide

The compound of interest was synthesized with a method similar to Step c in Example 1 by taking ((E)-7-(3-(3,4,5-trimethoxybenzal)-2,5-dioxopyrrolidinyl)ethyl heptanoate as a starting material. The compound of interest was a white solid, with the yield of 19.7%. 1H NMR (400 MHz, DMSO-d6) δ 10.33 (s, 1H), 8.65 (s, 1H), 7.43 (t, J = 2.4 Hz, 1H), 6.95 (s, 2H), 3.85 (s, 6H), 3.80 (d, J = 2.3 Hz, 2H), 3.71 (s, 3H), 3.48 (t, J = 7.1 Hz, 2H), 1.93 (t, J = 7.4 Hz, 2H), 1.59 - 1.43 (m, 4H), 1.31 - 1.20 (m, 4H).

13C NMR (101 MHz, DMSO) δ 174.89, 171.18, 169.52, 153.46, 139.44, 132.76, 130.11, 124.57, 108.23, 60.59, 56.46, 38.46, 33.85, 32.63, 28.62, 27.67, 26.45, 25.46.

### Example 36

### (E)-7-(3-(4-hydroxy-3,5-dimethoxybenzal)-2,5-diketopyrrolidinyl)-N-hydroxyheptami de (I-36)

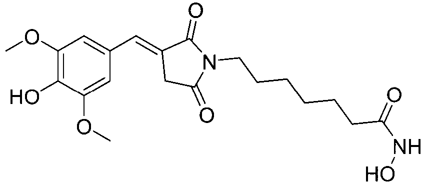

### Step a: Synthesis of (E)-3-(4-hydroxy-3,5-dimethoxybenzal)pyrrolidin-2,5-dione

The compound of interest was synthesized with a method similar to Step a in Example 1 by taking 4-hydroxy-3,5-dimethoxybenzaldehyde as a starting material.

### Step b: Synthesis of (E)-7-(3-(4-hydroxy-3,5-dimethoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-hydroxy-3,5-dimethoxybenzal)-2,5-dioxopyrrolidinyl)-N-hydroxyheptamide

The compound of interest was synthesized with a method similar to Step c in Example 1 by taking ((E)-7-(3-(4-hydroxy-3,5-dimethoxybenzal)-2,5-dioxopyrrolidinyl)ethyl heptanoate as a starting material. The compound of interest was a yellow solid, with the yield of 13.1%. 1H NMR (400 MHz, DMSO-d6) δ 10.32 (s, 1H), 9.06 (s, 1H), 8.65 (s, 1H), 7.39 (s, 1H), 6.91 (s, 2H), 3.82 (s, 6H), 3.75 (s, 2H), 3.48 (d, J = 7.2 Hz, 2H), 1.92 (t, J = 7.3 Hz, 2H), 1.52 - 1.44 (m, 4H), 1.27 - 1.21 (m, 4H).

13C NMR (101 MHz, DMSO) δ 174.98, 171.35, 169.52, 148.46, 138.40, 133.48, 124.91, 122.02, 108.63, 56.51, 38.38, 33.93, 32.64, 28.62, 27.71, 26.45, 25.46.

### Example 37

### (E)-7-(3-(2-hydroxybenzal)-2,5-diketopyrrolidinyl)-N-hydroxyheptamide (I-37)

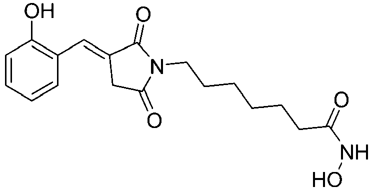

### Step a: Synthesis of (E)-3-(2-hydroxybenzal)pyrrolidin-2,5-dione

The compound of interest was synthesized with a method similar to Step a in Example 1 by taking 2-hydroxybenzaldehyde as a starting material.

### Step b: Synthesis of (E)-7-(3-(2-hydroxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-hydroxybenzal)-2,5-dioxopyrrolidinyl)-N-hydroxyheptamide

The compound of interest was synthesized with a method similar to Step c in Example 1 by taking ((E)-7-(3-(2-hydroxybenzal)-2,5-dioxopyrrolidinyl)ethyl heptanoate as a starting material. The compound of interest was a white solid, with the yield of 21.2%. 1H NMR (400 MHz, DMSO-d6) δ 10.33 (s, 1H), 10.20 (s, 1H), 8.65 (s, 1H), 7.80 (t, J = 2.5 Hz, 1H), 7.49 (d, J = 7.9 Hz, 1H), 7.25 (t, J = 7.6 Hz, 1H), 6.94 (d, J = 8.1 Hz, 1H), 6.88 (t, J = 7.5 Hz, 1H), 3.65 (d, J = 2.7 Hz, 2H), 3.47 (t, J = 7.1 Hz, 2H), 1.93 (t, J = 7.3 Hz, 2H), 1.62 - 1.42 (m, 4H), 1.34 - 1.18 (m, 4H).

13C NMR (101 MHz, DMSO) δ 174.94, 171.38, 169.54, 157.51, 131.82, 129.56, 127.17, 123.88, 121.49, 119.87, 116.31, 38.41, 34.05, 32.64, 28.63, 27.70, 26.45, 25.45.

### Example 38

### (E)-7-(3-(2-ethoxybenzal)-2,5-diketopyrrolidinyl)-N-hydroxyheptamide (I-38)

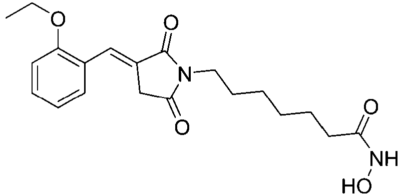

### Step a: Synthesis of (E)-3-(2-ethoxybenzal)pyrrolidin-2,5-dione

The compound of interest was synthesized with a method similar to Step a in Example 1 by taking 2-ethoxybenzaldehyde as a starting material.

### Step b: Synthesis of (E)-7-(3-(2-ethoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(2-ethoxybenzal)-2,5-dioxopyrrolidinyl)-N-hydroxyheptamide

The compound of interest was synthesized with a method similar to Step c in Example 1 by taking ((E)-7-(3-(2-ethoxybenzal)-2,5-dioxopyrrolidinyl)ethyl heptanoate as a starting material. The compound of interest was a white solid, with the yield of 12.5%. 1H NMR (300 MHz, DMSO-d6) δ 10.35 (s, 1H), 8.68 (s, 1H), 7.79 (t, J = 2.3 Hz, 1H), 7.57 (dd, J = 7.8, 1.6 Hz, 1H), 7.41 (ddd, J = 8.8, 7.4, 1.6 Hz, 1H), 7.10 (dd, J = 8.4, 1.0 Hz, 1H), 7.02 (t, J = 7.5 Hz, 1H), 4.13 (q, J = 6.9 Hz, 2H), 3.66 (d, J = 2.4 Hz, 2H), 3.47 (t, J = 7.1 Hz, 2H), 1.93 (t, J = 7.3 Hz, 2H), 1.55 - 1.44 (m, 4H), 1.38 (t, J = 6.9 Hz, 3H), 1.26 - 1.23 (m, 4H).

13C NMR (75 MHz, DMSO) δ 174.89, 171.25, 169.52, 157.82, 131.99, 129.62, 126.64, 125.13, 123.03, 121.03, 112.81, 64.23, 38.43, 34.00, 32.63, 28.63, 27.68, 26.44, 25.44, 15.07.

### Example 39

### (E)-7-(3-(5-bromo-2-methoxybenzal)-2,5-diketopyrrolidinyl)-N-hydroxyheptamide (I-39)

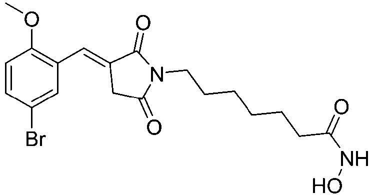

### Step a: Synthesis of (E)-3-(5-bromo-2-methoxybenzal)pyrrolidin-2,5-dione

The compound of interest was synthesized with a method similar to Step a in Example 1 by taking 5-bromo-2-methoxybenzaldehyde as a starting material.

### Step b: Synthesis of (E)-7-(3-(5-bromo-2-methoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(5-bromo-2-methoxybenzal)-2,5-dioxopyrrolidinyl)-N-hydroxyheptamide

The compound of interest was synthesized with a method similar to Step c in Example 1 by taking ((E)-7-(3-(5-bromo-2-methoxybenzal)-2,5-dioxopyrrolidinyl)ethyl heptanoate as a starting material. The compound of interest was a white solid, with the yield of 19.8%. 1H NMR (400 MHz, DMSO-d6) δ 10.33 (s, 1H), 8.65 (s, 1H), 7.65 (t, J = 2.4 Hz, 1H), 7.50 (d, J = 8.4 Hz, 1H), 7.33 (d, J = 1.9 Hz, 1H), 7.23 (dd, J = 8.3, 1.9 Hz, 1H), 3.90 (s, 3H), 3.63 (d, J = 2.5 Hz, 2H), 3.46 (t, J = 7.2 Hz, 2H), 1.93 (t, J = 7.4 Hz, 2H), 1.56 - 1.42 (m, 4H), 1.27 - 1.22 (m, 4H).

13C NMR (101 MHz, DMSO) δ 174.72, 171.06, 169.52, 159.07, 130.97, 126.17, 125.42, 124.82, 124.10, 122.34, 115.37, 56.79, 38.48, 33.96, 32.64, 28.62, 27.65, 26.43, 25.44.

### Example 40

### (E)-7-(3-(4-bromo-2-methoxybenzal)-2,5-diketopyrrolidinyl)-N-hydroxyheptamide (I-40)

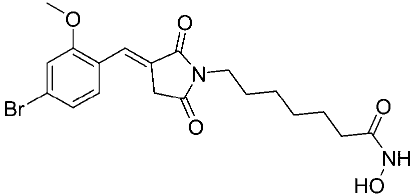

### Step a: Synthesis of (E)-3-(4-bromo-2-methoxybenzal)pyrrolidin-2,5-dione

The compound of interest was synthesized with a method similar to Step a in Example 1 by taking 4-bromo-2-methoxybenzaldehyde as a starting material.

### Step b: Synthesis of (E)-7-(3-(4-bromo-2-methoxybenzal)-2,5-dioxopyrrolidinyl)ethyl heptanoate

The compound of interest was synthesized with a method similar to Step b in Example 1 by taking ethyl bromoheptanoate as a starting material.

### Step c: Synthesis of (E)-7-(3-(4-bromo-2-methoxybenzal)-2,5-dioxopyrrolidinyl)-N-hydroxyheptamide

The compound of interest was synthesized with a method similar to Step c in Example 1 by taking ((E)-7-(3-(4-bromo-2-methoxybenzal)-2,5-dioxopyrrolidinyl)ethyl heptanoate as a starting material. The compound of interest was a white solid, with the yield of 18.6%. 1H NMR (400 MHz, DMSO-d6) δ 10.33 (s, 1H), 8.65 (s, 1H), 7.67 - 7.63 (m, 2H), 7.60 (dd, J = 8.8, 2.4 Hz, 1H), 7.10 (d, J = 8.9 Hz, 1H), 3.88 (s, 3H), 3.70 (d, J = 2.5 Hz, 2H), 3.47 (t, J = 7.2 Hz, 2H), 1.93 (t, J = 7.3 Hz, 2H), 1.56 - 1.42 (m, 4H), 1.29 - 1.19 (m, 4H).

13C NMR (101 MHz, DMSO) δ 174.70, 170.92, 169.52, 157.63, 134.22, 131.44, 127.04, 125.22, 125.12, 114.33, 112.73, 56.60, 38.50, 33.64, 32.64, 28.61, 27.64, 26.42, 25.44.

### Example 41

### (E)-4-((3-benzal-2,5-diketopyrrolidinyl)methyl)-N-hydroxybenzamide (I-41)

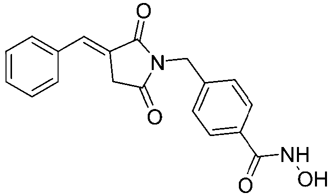

### Step a: Synthesis of (E)-3-benzalpyrrolidin-2,5-dione

The operation and feeding were the same as Step a in Example 1.

### Step b: Synthesis of (E)-4-((3-benzal-2,5-diketopyrrolidinyl)methyl)ethyl benzoate

The compound of interest was synthesized with a method similar to Step b in Example 1 by taking ethyl 4-(bromomethyl)benzoate as a starting material.

### Step c: Synthesis of (E)-4-((3-benzal-2,5-diketopyrrolidinyl)methyl)-N-hydroxybenzamide

The compound of interest was synthesized with a method similar to Step c in Example 1 by taking (E)-4-((3-benzal-2,5-diketopyrrolidinyl)methyl)ethyl benzoate as a starting material. The compound of interest was a yellow solid, with the yield of 11.3%. 1H NMR (400 MHz, DMSO-d6) δ 11.18 (s, 1H), 9.12 (s, 1H), 7.71 (d, J = 7.9 Hz, 2H), 7.67 (d, J = 7.0 Hz, 2H), 7.54 - 7.43 (m, 4H), 7.36 (d, J = 7.9 Hz, 2H), 4.74 (s, 2H), 3.82 (d, J = 2.3 Hz, 2H).

13C NMR (101 MHz, DMSO) δ 174.67, 170.98, 164.41, 139.79, 134.50, 132.98, 132.38, 130.77, 130.41, 129.49, 127.84, 127.56, 125.62, 41.69, 34.34.

Pharmacological tests were conducted on the compounds obtained in the above examples, with the results listed as follows.

### (1) HDAC inhibitory activity assay and results of compounds of interest

The HDAC inhibitory activity of the synthesized compounds was determined by fluorescence resonance energy transfer (FRET), and then compared with that of the positive control to screen out compounds with better activity. HDAC was obtained by purification or purchased directly.

The method was as follows.

An enzyme was added to reaction wells, a reaction buffer was added to control wells, a sample dissolved in DMSO was added to the reaction wells, and the wells were incubated using a contactless nanoflow sonic pipetting system (Ech-550; nanoflow). A fluorescent substrate was added to each reaction well and then rotated and shaken. The wells were incubated for 1-2 h at 30°C under a sealed condition. A TMP26-containing chromogenic agent was added to terminate the reaction and produce fluorescence. The fluorescence intensity was detected using EnVision multi-label microplate detector (Perkin Elmer) (excitation light: 490 nm, emitted light: 520 nm). After the stabilization of color development, the endpoint value was read. The percentage (relative to the DMSO control group) inhibition rate was calculated using the software GraphPad Prism 4.

Results of HDAC inhibitory activity of compounds of itnerest:

| Example | Test concentration (nM) | Inhibition rate |
|---|---|---|
| **I-3** | 100 | 77.8% ± 1.2% |
| **I-5** | 100 | 84.8% ± 2.1% |
| **1-7** | 100 | 0 |
| **I-8** | 100 | 5.3% ± 0.2% |
| **I-12** | 100 | 0 |
| **I-13** | 100 | 0 |
| **I-14** | 100 | 0 |
| **I-15** | 100 | 81.4% ± 0.5% |
| **I-16** | 100 | 93.3% ± 1.0% |
| **I-17** | 100 | 76.2% ± 0.7% |
| **I-18** | 100 | 85.9% ± 0.9% |
| **I-19** | 100 | 84.5% ± 0.2% |
| **I-21** | 100 | 88.3% ± 1.1% |
| **I-22** | 100 | 89.4% ± 0.9% |
| **I-23** | 100 | 84.9% ± 0.5% |
| **I-25** | 100 | 91.2% ± 0.7% |
| **I-26** | 100 | 86.6% ± 0.4% |
| **I-27** | 100 | 90.9% ± 1.2% |
| **I-28** | 100 | 85.2% ± 1.6% |
| **I-29** | 100 | 91.3% ± 0.7% |
| **I-30** | 100 | 82.1% ± 0.1% |
| **I-34** | 100 | 85.3% ± 7.2% |
| **I-35** | 100 | 82.5% ± 3.5% |
| **I-36** | 100 | 74.8% ± 2.8% |
| **I-37** | 100 | 90.6% ± 0.9% |
| **I-39** | 100 | 88.1% ± 1.1% |
| **I-41** | 100 | 77.1% ± 1.8% |

| Compd. | Half-maximal inhibitory concentration (IC50 ± SD, nM) |
|---|---|
| **I-4** | 12.9 ± 0.10 |
| **I-20** | 40.1 ± 1.21 |
| **I-24** | 19.3 ± 0.11 |
| **I-31** | 12.6 ± 0.15 |
| **I-32** | 18.5 ± 0.19 |
| **I-33** | 13.0 ± 0.02 |
| **I-38** | 16.7 ± 0.21 |
| **I-40** | 5.50 ± 0.03 |

### (2) In vitro anti-tumor activity assay of compounds of interest

The inhibitory effects of the compounds against human non-small cell lung cancer cell lines A549, NCI-H1650, human small cell lung cancer cell lines H446, human prostate cancer cells DU145, PC-3, LNCAP, human colon cancer cells lines HCT116, human breast cancer cells MDA-MB-231, and MCF7 tumor cell lines were determined using the MTT method.

The method was as follows. The cells in the exponential growth phase were collected to count living cells. The concentration of the cell suspension was adjusted using the corresponding medium of each group of cells. The cell suspension was added to the 96-well culture plate at 100 µL per cell, and the plate was placed in a 5% CO₂ incubator at 37°C and incubated for 24 h. Each test compound was dissolved in DMSO to prepare a stock solution with a corresponding concentration. Then, each stock solution was diluted 500-fold with the medium to prepare 4 replicate wells. 100 µL of the corresponding 500-fold solution was added to each well per cell line, with the final DMSO concentration of 0.1%. After 96-hour treatment, 10 µL of 5 mg/mL solution of MTT in PBS was added to each well; the plate was placed in the 5% CO₂ incubator at 37°C for treatment for 4 h; then, the medium in the 96-well plate was removed, and 150 mL of DMSO was added to each well; and the absorbance was detected on a microplate reader. The half-maximal (relative to the DMSO control group) inhibition rate was calculated using the software GraphPad Prism 4.

The results of the in vitro anti-tumor activity (IC₅₀) (in µM) of the compounds of interest against the tumor cell lines described above were shown below.

| Example | In vitro anti-tumor activity (IC₅₀) | | | |
|---|---|---|---|---|
| | DU145 | PC-3 | A549 | MCF-7 |
| **I-1** | > 20 | > 20 | > 20 | > 20 |
| **I-2** | > 20 | > 20 | > 20 | > 20 |
| **I-3** | 1.49 ± 0.21 | 4.27 ± 0.25 | 3.51 ± 0.15 | 2.58 ± 0.07 |
| **I-4** | 0.50 ± 0.07 | 0.82 ± 0.07 | 1.32 ± 0.17 | 1.43 ± 0.06 |
| **I-5** | 1.56 ± 0.18 | 5.01 ± 0.38 | 5.88 ± 0.58 | 3.89 ± 0.36 |
| **I-6** | > 20 | > 20 | > 20 | > 20 |
| **I-7** | > 20 | > 20 | > 20 | > 20 |
| **I-8** | 11.07 ± 0.34 | > 20 | 17.33 ± 2.19 | > 20 |
| **I-9** | > 20 | > 20 | > 20 | > 20 |
| **I-10** | > 20 | > 20 | > 20 | > 20 |
| **I-11** | > 20 | > 20 | > 20 | > 20 |
| **I-12** | > 20 | > 20 | > 20 | > 20 |
| **I-13** | > 20 | > 20 | > 20 | > 20 |
| **I-14** | > 20 | > 20 | > 20 | > 20 |
| **I-15** | 1.35 ± 0.27 | 1.82 ± 0.16 | 3.68 ± 0.50 | 1.66 ± 0.14 |
| **I-16** | 0.92 ± 0.03 | 1.75 ± 0.12 | 2.24 ± 0.08 | 1.16 ± 0.12 |
| **I-17** | 1.22 ± 0.18 | 1.83 ± 0.05 | 3.37 ± 0.43 | 1.68 ± 0.09 |
| **I-18** | 0.31 ± 0.05 | 2.20 ± 0.08 | 1.88 ± 0.17 | 0.46 ± 0.04 |
| **I-19** | 0.71 ± 0.05 | 0.93 ± 0.06 | 3.48 ± 0.22 | 1.41 ± 0.11 |
| **I-20** | 0.74 ± 0.10 | 0.87 ± 0.10 | 2.82 ± 0.39 | 0.82 ±0.05 |
| **I-21** | 0.67 ± 0.09 | 0.99 ± 0.04 | 2.25 ± 0.25 | 1.54 ± 0.09 |
| **I-22** | 0.34 ± 0.03 | 1.29 ± 0.14 | 1.80 ± 0.14 | 0.98 ± 0.02 |
| **I-23** | 0.54 ± 0.10 | 0.91 ± 0.07 | 3.44 ± 0.15 | 0.97 ± 0.05 |
| **I-24** | 0.48 ± 0.06 | 1.44 ± 0.10 | 1.33 ± 0.23 | 0.58 ± 0.03 |
| **I-25** | 0.38 ± 0.04 | 1.70 ± 0.08 | 1.12 ± 0.20 | 1.05 ± 0.24 |
| **I-26** | 0.57 ± 0.04 | 0.89 ± 0.12 | 2.20 ± 0.26 | 0.69 ± 0.09 |
| **I-27** | 0.65 ± 0.09 | 1.06 ± 0.13 | 2.81 ± 0.10 | 0.83 ± 0.05 |
| **I-28** | 0.66 ± 0.12 | 0.89 ± 0.05 | 3.02 ± 0.51 | 0.92 ± 0.05 |
| **I-29** | 0.91 ± 0.07 | 1.47 ± 0.15 | 5.09 ± 0.23 | 1.38 ± 0.12 |

| Example | In vitro anti-tumor activity (IC₅₀) | | | |
|---|---|---|---|---|
| | DU145 | PC-3 | A549 | MCF-7 |
| **I-30** | 0.44 ± 0.08 | 1.69 ± 0.08 | 2.34 ± 0.10 | 0.90 ± 0.02 |
| **I-31** | 0.16 ± 0.04 | 0.82 ± 0.09 | 1.39 ± 0.06 | 0.35 ± 0.02 |
| **I-32** | 0.40 ± 0.04 | 0.92 ± 0.03 | 1.24 ± 0.21 | 0.88 ± 0.09 |
| **I-33** | 0.50 ± 0.10 | 0.89 ± 0.10 | 1.52 ± 0.14 | 1.02 ± 0.11 |
| **I-34** | 0.45 ± 0.12 | 0.88 ± 0.10 | 1.23 ± 0.17 | 0.82 ± 0.09 |
| **I-35** | 1.03 ± 0.14 | 1.71 ± 0.17 | 3.63 ± 0.65 | 1.27 ± 0.13 |
| **I-36** | 3.88 ± 0.04 | 1.73 ± 0.20 | > 10 | 1.88 ± 0.14 |
| **I-37** | 1.72 ± 0.12 | 1.93 ± 0.23 | 4.34 ± 0.69 | 0.80 ± 0.15 |
| **I-38** | 0.34 ± 0.04 | 0.89 ± 0.05 | 1.78 ± 0.2 | 0.51 ± 0.07 |
| **I-39** | 0.46 ± 0.04 | 1.13 ± 0.07 | 2.21 ± 0.27 | 0.68 ± 0.09 |
| **I-40** | 0.25 ± 0.04 | 0.67 ± 0.10 | 1.49 ± 0.09 | 0.32 ± 0.02 |
| **I-41** | 2.54 ± 0.41 | 7.82 ± 0.90 | 3.09 ± 0.41 | 6.97 ± 0.83 |

| Example | Antiproliferative Activity (IC50 ± SD, µM) | | | | | |
|---|---|---|---|---|---|---|
| | MDA-MB-231 | HCT116 | HT29 | H446 | LNCAP | H1650 |
| **I-4** | 0.67 ± 0.10 | 0.75 ± 0.08 | 1.76 ± 0.25 | 0.57 ± 0.03 | 1.63 ± 0.24 | 6.12 ± 0.88 |
| **I-22** | 0.98 ± 0.11 | 0.91 ± 0.11 | 1.69 ± 0.10 | 0.70 ± 0.10 | 1.48 ± 0.20 | 4.42 ± 0.72 |
| **I-24** | 0.99 ± 0.11 | 0.86 ± 0.08 | 1.22 ± 0.10 | 0.77 ± 0.10 | 1.51 ± 0.22 | 6.60 ±0.57 |
| **I-25** | 2.05 ± 0.20 | 0.81 ± 0.06 | 1.30 ± 0.10 | 0.78 ± 0.04 | 1.95 ± 0.21 | 7.30 ± 0.56 |
| **I-31** | 0.31 ± 0.05 | 0.47 ± 0.01 | 1.05 ± 0.10 | 0.65 ± 0.05 | 0.63 ± 0.12 | 3.56 ± 0.10 |

The above pharmacological test results indicate that the compounds of formula I of the present invention and the pharmaceutically acceptable salts thereof exhibit excellent inhibitory activity against HDACs, and thus can be used for treating the clinical diseases related to the target described above. The HDAC-related clinical disease may include, but not limited to, lung cancer, melanoma, liver cancer, kidney cancer, leukemia, non-small cell lung cancer, prostate cancer, thyroid cancer, skin cancer, pancreatic cancer, ovarian cancer, testicular cancer, breast cancer, bladder cancer, gallbladder cancer, myelodysplastic syndrome, lymphoma, esophageal cancer, follicular thyroid carcinoma, gastrointestinal cancer, central or peripheral nervous system tumor (e.g., astrocytoma, neuroblastoma, glioma or schwannoma), mesothelioma, type II or non-insulin-dependent diabetes mellitus, and autoimmune disease.

The present invention may also include other embodiments in addition to those described above. Any technical solution achieved by equivalent substitution or equivalent transformation shall fall within the protection scope claimed by the present invention.

## Claims

1. A compound of formula (I) or a pharmaceutically acceptable salt thereof, 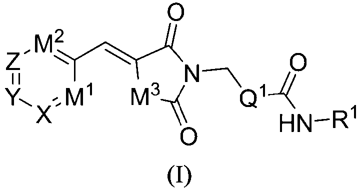
wherein in the formula, X, Y, Z, M¹, and M² each independently represent a carbon or nitrogen atom;
in a case where X, Y, Z, M¹, and M² each independently represent the carbon atom, X, Y, Z, M¹, and M² are each independently and optionally substituted by R², with R² being hydrogen, alkyl, cyano, halogen, haloalkyl, hydroxy, mercapto, alkoxy, alkylamino, alkylthio, alkoxyalkyl, arylalkyl, diarylalkyl, aryl, or Het;
M³ is carbon and is independently and optionally substituted by R⁴ or R⁵, with R⁴ or R⁵ being hydrogen, deuterium, alkyl, haloalkyl, hydroxy, mercapto, alkoxy, alkylamino, alkylthio, alkoxyalkyl, methylene, arylalkyl, diarylalkyl, aryl, or Het;
Q¹ is selected from saturated or unsaturated linear or branched hydrocarbonyl, aryl, or Het with a length of 1-8 carbon atoms;
R¹ is selected from hydroxy, 2-aminophenyl substituted by one or more R³, or 2-hydroxycyclohexyl;
R³ is hydrogen, alkyl, cyano, halogen, haloalkyl, hydroxy, mercapto, alkoxy, alkylthio, alkoxyalkyl, arylalkyl, aryl, or Het;
the alkyl is linear or branched saturated hydrocarbonyl with 1-7 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms;
the alkoxy is linear or branched saturated hydrocarbonyl with 1-6 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms, wherein each carbon atom is optionally substituted by oxygen;
the alkylamino is linear or branched saturated hydrocarbonyl with 1-6 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms, wherein each carbon atom is optionally substituted by an NH atomic group;
the alkoxyalkyl is derived by linking the alkoxy to the alkyl as defined above;
alkenyl or alkynyl is linear or branched doubled-bonded or triple-bonded unsaturated hydrocarbonyl with 1-6 carbon atoms;
the aryl is a carbocyclic ring selected from phenyl, naphthyl, acenaphthenyl, or tetrahydronaphthyl, and is optionally substituted by 1, 2, or 3 substituents, with each substituent independently selected from hydrogen, alkyl, cyano, halogen, haloalkyl, hydroxy, mercapto, alkoxy, alkylthio, alkoxyalkyl, arylalkyl, diarylalkyl, aryl, or Het;
the arylalkyl or the diarylalkyl is derived by linking the aryl to the alkyl as defined above;
the Het is a monocyclic heterocyclic ring selected from pyrrolyl, pyrazolyl, imidazolyl, furyl, thienyl, oxazolyl, isooxazolyl, thiazolyl, isothiazolyl, pyridyl, pyrimidinyl, pyrazinyl, or pyridazinyl, or a bicyclic heterocyclic ring selected from quinolyl, quinoxalinyl, indolyl, benzoimidazolyl, benzoxazolyl, benzoisooxazolyl, benzothiazolyl, benzoisothiazolyl, benzofuryl, benzothienyl, 2, 3-dihydrobenzo[1,4]dioxacyclohexenyl, or benzo[1,3]dioxacyclopentenyl, or is selected from monocyclic saturated hydrocarbonyl with 3-6 carbon atoms, or bicyclic saturated hydrocarbonyl with 6-12 carbon atoms, wherein the carbon atom in the ring is independently substituted by 1-4 O, S, N, or NH, each monocyclic or bicyclic ring is optionally substituted by 1, 2, or 3 substituents, and each substituent is independently selected from halogen, haloalkyl, hydroxy, alkyl, or alkoxy;
the halogen is a substituent selected from fluorine, chlorine, bromine, or iodine; and
the haloalkyl is linear or branched saturated hydrocarbonyl with 1-6 carbon atoms, or cyclic saturated hydrocarbonyl with 3-6 carbon atoms, or cyclic saturated hydrocarbonyl with 3-6 carbon atoms that is linked to linear or branched saturated hydrocarbonyl with 1-6 carbon atoms, wherein one or more carbon atoms are optionally substituted by one or more halogen atoms.

2. The compound according to claim 1, wherein in the formula, X, Y, Z, M¹, and M² each independently represent a carbon or nitrogen atom, and in a case where X, Y, Z, M¹, and M² each independently represent the carbon atom, X, Y, Z, M¹, and M² are each independently and optionally substituted by R², with R² being hydrogen, alkyl, cyano, halogen, haloalkyl, alkoxy, alkylthio, or alkoxyalkyl;
M³ is carbon and is independently and optionally substituted by R⁴ or R⁵, with R⁴ or R⁵ being hydrogen, deuterium, alkyl, haloalkyl, hydroxy, mercapto, alkoxy, alkylamino, alkylthio, alkoxyalkyl, methylene, arylalkyl, diarylalkyl, aryl, or Het;
Q¹ is selected from saturated or unsaturated linear or branched hydrocarbonyl or aryl with a length of 1-8 carbon atoms;
R¹ is selected from hydroxy, or 2-aminophenyl optionally substituted by one or more R³; and
R³ is hydrogen, alkyl, cyano, halogen, haloalkyl, aryl, or Het.

3. The compound according to claim 1, wherein in the formula, X, Y, Z, M¹, and M² each independently represent a carbon or nitrogen atom, and in a case where X, Y, Z, M¹, and M² each independently represent the carbon atom, X, Y, Z, M¹, and M² are each independently and optionally substituted by R², with R² being hydrogen, alkyl, halogen, or alkoxy;
M³ is carbon and is independently and optionally substituted by R⁴ or R⁵, with R⁴ or R⁵ being hydrogen, deuterium, alkyl, haloalkyl, hydroxy, mercapto, alkoxy, alkylamino, alkylthio, alkoxyalkyl, methylene, arylalkyl, diarylalkyl, aryl, or Het;
Q¹ is selected from saturated or unsaturated linear or branched hydrocarbonyl or aryl with a length of 3-8 carbon atoms;
R¹ is selected from hydroxy, or 2-aminophenyl optionally substituted by one or more R³; and
R³ is hydrogen, alkyl, cyano, halogen, haloalkyl, aryl, or Het.

4. The compound according to claim 1, wherein the compound is:
(E)-4-(3-benzal-2,5-dioxopyrrolidinyl)-N-hydroxybutyramide (I-1),
(E)-4-(3-benzal-2,5-dioxopyrrolidinyl)-N-hydroxypentanamide (I-2),
(E)-4-(3-benzal-2,5-dioxopyrrolidinyl)-N-hydroxyhexanamide (I-3),
(E)-4-(3-benzal-2,5-dioxopyrrolidinyl)-N-hydroxyheptamide (I-4),
(E)-4-(3-benzal-2,5-dioxopyrrolidinyl)-N-hydroxydecoylamide (I-5),
(E)-N-(2-amino-4-fluorophenyl)-4-(3-benzal-2,5-diketopyrrolidinyl)butyramide (I-6),
(E)-N-(2-amino-4-fluorophenyl)-5-(3-benzal-2,5-diketopyrrolidinyl)pentanamide (I-7),
(E)-N-(2-amino-4-fluorophenyl)-6-(3-benzal-2,5-diketopyrrolidinyl)hexanamide (I-8),
(E)-N-(2-amino-4-fluorophenyl)-7-(3-benzal-2,5-diketopyrrolidinyl)heptamide (I-9),
4-(3-((E)-benzal)-2,5-diketopyrrolidinyl)-N-((1S,2S)-2-hydroxycyclohexyl)butyramide (I-10),
5-(3-((E)-benzal)-2,5-diketopyrrolidinyl)-N-((1S,2S)-2-hydroxycyclohexyl)pentanamide (I-11),
6-(3-((E)-benzal)-2,5-diketopyrrolidinyl)-N-((1S,2S)-2-hydroxycyclohexyl)hexanamide (I-12),
7-(3-((E)-benzal)-2,5-diketopyrrolidinyl)-N-((1S,2S)-2-hydroxycyclohexyl) heptamide (I-13),
8-(3-((E)-benzal)-2,5-diketopyrrolidinyl)-N-((1S,2S)-2-hydroxycyclohexyl)decoylamide (I-14),
(E)-7-(3-(4-isopropylbenzal)-2,5-diketopyrrolidinyl)-N-hydroxyheptamide (I-15),
(E)-7-(3-(4-dimethylaminobenzal)-2,5-diketopyrrolidinyl)-N-hydroxyheptamide (I-16),
(E)-7-(3-(4-trifluoromethylbenzal)-2,5-diketopyrrolidinyl)-N-hydroxyheptamide (I-17),
(E)-7-(3-(4-methylbenzal)-2,5-diketopyrrolidinyl)-N-hydroxyheptamide (I-18),
(E)-7-(3-(2-methylbenzal)-2,5-diketopyrrolidinyl)-N-hydroxyheptamide (I-19),
(E)-7-(3-(2-cyanobenzal)-2,5-diketopyrrolidinyl)-N-hydroxyheptamide (I-20),
(E)-7-(3-(2-methylenepyridin)-2,5-diketopyrrolidinyl)-N-hydroxyheptamide (I-21),
(E)-7-(3-(2-methylenenaphthyl)-2,5-diketopyrrolidinyl)-N-hydroxyheptamide (I-22),
(E)-7-(3-(2-methylenethienyl)-2,5-diketopyrrolidinyl)-N-hydroxyheptamide (I-23),
(E)-7-(3-(2-chlorobenzal)-2,5-diketopyrrolidinyl)-N-hydroxyheptamide (I-24),
(E)-7-(3-(3-chlorobenzal)-2,5-diketopyrrolidinyl)-N-hydroxyheptamide (I-25),
(E)-7-(3-(4-chlorobenzal)-2,5-diketopyrrolidinyl)-N-hydroxyheptamide (I-26),
(E)-7-(3-(3-bromobenzal)-2,5-diketopyrrolidinyl)-N-hydroxyheptamide (I-27),
(E)-7-(3-(3-fluorobenzal)-2,5-diketopyrrolidinyl)-N-hydroxyheptamide (I-28),
(E)-7-(3-(3-iodobenzal)-2,5-diketopyrrolidinyl)-N-hydroxyheptamide (I-29),
(E)-7-(3-(3,4-dichlorobenzal)-2,5-diketopyrrolidinyl)-N-hydroxyheptamide (I-30),
(E)-7-(3-(2-methoxybenzal)-2,5-diketopyrrolidinyl)-N-hydroxyheptamide (I-31),
(E)-7-(3-(3-methoxybenzal)-2,5-diketopyrrolidinyl)-N-hydroxyheptamide (I-32),
(E)-7-(3-(4-methoxybenzal)-2,5-diketopyrrolidinyl)-N-hydroxyheptamide (I-33),
(E)-7-(3-(3,4-dimethoxybenzal)-2,5-diketopyrrolidinyl)-N-hydroxyheptamide (I-34),
(E)-7-(3-(3,4,5-trimethoxybenzal)-2,5-diketopyrrolidinyl)-N-hydroxyheptamide (I-35),
(E)-7-(3-(4-hydroxy-3,5-dimethoxybenzal)-2,5-diketopyrrolidinyl)-N-hydroxyheptamide (I-36),
(E)-7-(3-(2-hydroxybenzal)-2,5-diketopyrrolidinyl)-N-hydroxyheptamide (I-37),
(E)-7-(3-(2-ethoxybenzal)-2,5-diketopyrrolidinyl)-N-hydroxyheptamide (I-38),
(E)-7-(3-(5-bromo-2-methoxybenzal)-2,5-diketopyrrolidinyl)-N-hydroxyheptamide (I-39),
(E)-7-(3-(4-bromo-2-methoxybenzal)-2,5-diketopyrrolidinyl)-N-hydroxyheptamide (I-40), or
(E)-4-((3-benzal-2,5-diketopyrrolidinyl)methyl)-N-hydroxybenzamide (I-41).

5. A pharmaceutical composition, comprising the compound according to any one of claims 1 to 4 or the pharmaceutically acceptable salt thereof, and a pharmaceutically acceptable carrier, wherein the compound according to any one of claims 1 to 4 or the pharmaceutically acceptable salt thereof is an active ingredient.

6. A use of the compound according to any one of claims 1 to 4 in preparation of a medicament for preventing or treating an HDAC-related clinical disease.

7. The use of the compound according to claim 6, wherein the HDAC-related clinical disease comprises lung cancer, melanoma, liver cancer, kidney cancer, leukemia, prostate cancer, thyroid cancer, skin disease, pancreatic cancer, ovarian cancer, testicular cancer, breast cancer, bladder cancer, gallbladder cancer, myelodysplastic syndrome, lymphoma, esophageal cancer, gastrointestinal cancer, astrocytoma, neuroblastoma, glioma, schwannoma, mesothelioma, non-insulin-dependent diabetes mellitus, or autoimmune disease.
